(19) Europäisches Patentamt
European Patent Office
Office européen des brevets

(11) Veröffentlichungsnummer : **0 345 629 B1**

(12) # EUROPÄISCHE PATENTSCHRIFT

(45) Veröffentlichungstag der Patentschrift : **16.09.92 Patentblatt 92/38**

(21) Anmeldenummer : **89109892.3**

(22) Anmeldetag : **01.06.89**

(51) Int. Cl.$^5$ : **C07D 471/04,** C07D 401/06, C07D 403/06, C07D 487/04, C07D 495/04, C07D 411/06, C07D 417/06, C07D 495/14, C07D 471/14, C07D 487/14, A61K 31/55, // (C07D471/04, 243:00, 221:00)

(54) **Kondensierte Diazepinone, Verfahren zu ihrer Herstellung und diese Verbindungen enthaltende Arzneimittel.**

Die Akte enthält technische Angaben, die nach dem Eingang der Anmeldung eingereicht wurden und die nicht in dieserPatentschrift enthalten sind.

(30) Priorität : **08.06.88 DE 3819444**

(43) Veröffentlichungstag der Anmeldung : **13.12.89 Patentblatt 89/50**

(45) Bekanntmachung des Hinweises auf die Patenterteilung : **16.09.92 Patentblatt 92/38**

(84) Benannte Vertragsstaaten : **AT BE CH DE ES FR GB GR IT LI LU NL SE**

(56) Entgegenhaltungen : **EP-A- 0 156 191**

(73) Patentinhaber : **Dr. Karl Thomae GmbH**
**Postfach 1755**
**W-7950 Biberach 1 (DE)**

(72) Erfinder : **Engel, Wolfhard, Dr. Dipl.-Chem.**
**Mozartstrasse 13**
**W-7950 Biberach 1 (DE)**
Erfinder : **Eberlein, Wolfgang, Dr. Dipl.-Chem.**
**Obere Au 6**
**W-7950 Biberach 1 (DE)**
Erfinder : **Mihm, Gerhard, Dr. Dipl.-Chem.**
**Nickeleshalde 5/1**
**W-7950 Biberach 1 (DE)**
Erfinder : **Trummlitz, Günter, Dr., Dipl.-Chem.**
**Buchenweg 27**
**W-7951 Warthausen (DE)**
Erfinder : **Mayer, Norbert, Dr.**
**Friedrich-Ebert-Strasse 66**
**W-7950 Biberach 1 (DE)**
Erfinder : **Doods, Henri, Dr.**
**Hornsteinweg 7**
**W-7951 Warthausen (DE)**
Erfinder : **de Jonge, Adriaan, Dr.**
**De Boomgaard 19**
**NL-3971 LD Driebergen (NL)**

**EP 0 345 629 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft kondensierte Diazepinone, Verfahren zu ihrer Herstellung und diese Verbindungen enthaltende Arzneimittel.

Aus EP-A-0 039 519 und 0 057 428 sowie aus US-A 3.660.380; 3.691.159; 4.213.984; 4.213.985; 4.210.648, 4.410.527; 4.424.225; 4.424.222 und 4.424.226 sind bereits kondensierte Diazepinone mit ulcushemmenden und magensaftsekretionshemmenden Eigenschaften bekannt.

In EP-A-0 156 191 ist beschrieben, daß durch Einführung neuartiger Aminoacylreste gegenüber den Verbindungen der obengenannten Publikationen völlig andersartige, wertvolle pharmakologische Eigenschaften induziert werden können. Gegenüber diesen Verbindungen zeichnen sich die erfindungsgemäßen kondensierten Diazepinone bei vergleichbarer oder verbesserter Selektivität und Resorption nach oraler Gabe durch eine wesentlich verstärkte Wirkung und ausgeprägte Hydrolysestabilität aus.

Die neuen kondensierten Diazepinone besitzen die allgemeine Formel I,

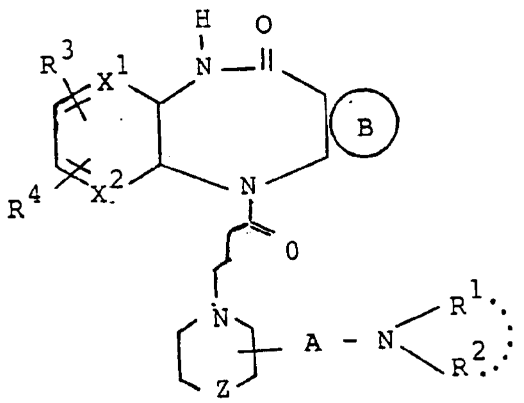

(I)

in der

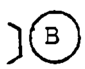 einen der zweiwertigen Reste

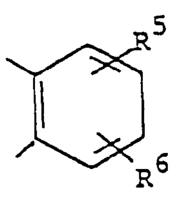 , 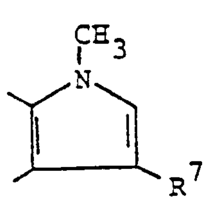 , 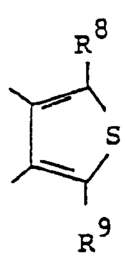 , 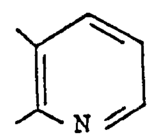 oder 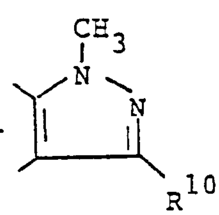

(S) (T) (U) (V) (W)

darstellt und

$X^1$, $X^2$, A, $R^1$ bis $R^{10}$ und Z die folgenden Bedeutungen besitzen:

$X^1$ und $X^2$ stellen eine =CH-Gruppe dar oder, sofern 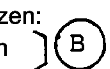 die Bedeutungen der oben genannten zweiwertigen Reste (S), (U) oder (W) annimmt, können beide oder nur $X^1$ oder nur $X^2$ auch ein Stickstoffatom darstellen,

A ist ein geradkettiger, gesättigter Alkylenrest mit 1 bis 6 Kohlenstoffatomen;

$R^1$ und $R^2$ sind gleiche oder voneinander verschiedene Alkylreste mit bis zu 6 Kohlenstoffatomen oder

bedeuten zusammen mit dem dazwischenliegenden Stickstoffatom einen 4- bis 7-gliedrigen, gesättigten, monocyclischen, heteroaliphatischen Ring, der gegebenenfalls durch ein Sauerstoffatom oder durch die N-$CH_3$-Gruppe unterbrochen sein kann;

$R^3$ ist eine Alkylgruppe mit I bis 4 C-Atomen, ein Chloratom oder ein Wasserstoffatom;

$R^4$ ein Wasserstoffatom oder eine Methylgruppe;

$R^5$ und $R^6$ sind jeweils ein Wasserstoffatom, ein Fluor-, Chlor- oder Bromatom oder eine Alkylgruppe mit I bis 4 C-Atomen;

$R^7$ ist ein Wasserstoff- oder Chloratom oder eine Methylgruppe;

$R^8$ ein Wasserstoffatom oder ein Alkylrest mit I bis 4 Kohlenstoffatomen;

$R^9$ ein Wasserstoff- oder Halogenatom oder eine Alkylgruppe mit I bis 4 Kohlenstoffatomen und $R^{I0}$ ein Wasserstoffatom oder eine Methylgruppe und
Z eine Einfachbindung, ein Sauerstoff- oder Schwefelatom, die Methylen- oder I,2-Ethylengruppe.

Für den Fall, daß 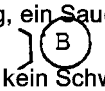 den zweiwertigen Rest (T) darstellt und $R^7$ ein Wasserstoffatom ist, kann $R^3$ kein Chloratom und Z kein Schwefelatom bedeuten.

Bevorzugte Verbindungen der obigen allgemeinen Formel I sind solche, in denen

$X^I$ eine =CH-Gruppe,

$X^2$ entweder ein Stickstoffatom und 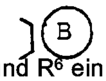 den zweiwertigen Rest (S) darstellt, mit der Maßgabe, daß $R^3$, $R^4$ und $R^5$ Wasserstoffatome sind und $R^6$ ein Wasserstoffatom, ein Chlor- oder Bromatom oder eine Methyl- oder Ethylgruppe in 8- oder 9-Stellung des Heterocyclus ist,

oder eine =CH-Gruppe darstellt, wenn 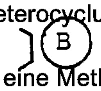 die Bedeutung der zweiwertigen Reste V oder U annimmt, wobei $R^8$ ein Wasserstoffatom und $R^9$ eine Methylgruppe ist;

A eine unverzweigte, gesättigte Alkylenkette mit I bis 5 Kohlenstoffatomen bedeutet;

$R^I$ und $R^2$ gleiche oder voneinander verschiedene Alkylreste mit I bis 5 Kohlenstoffatomen oder zusammen mit dem eingeschlossenen Stickstoffatom einen 4- bis 7-gliedrigen, gesättigten, monocyclischen, heteroaliphatischen Ring darstellen, der gegebenenfalls durch ein Sauerstoffatom oder durch die N-$CH_3$-Gruppe unterbrochen sein kann

und

Z eine Einfachbindung, ein Sauerstoffatom, die Methylen-oder I,2-Ethylengruppe ist.

Ganz besonders bevorzugt werden solche Verbindungen der allgemeinen Formel I, in der

$X^I$ eine =CH-Gruppe;

$X^2$ ein Stickstoffatom und 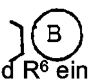 den zweiwertigen Rest (S) bedeutet, mit der Maßgabe, daß $R^3$, $R^4$ und $R^5$ Wasserstoffatome sind und $R^6$ ein Wasserstoffatom, ein Chloroder Bromatom oder eine Methyl- oder Ethylgruppe in 8- oder 9-Stellung des Heterocyclus ist,

A eine unverzweigte, gesättigte Alkylenkette mit I bis 5 Kohlenstoffatomen bedeutet,

$R^I$ und $R^2$ gleiche oder voneinander verschiedene Alkylreste mit I bis 5 Kohlenstoffatomen oder zusammen mit dem dazwischenliegenden Stickstoffatom einen 5- bis 7-gliedrigen, gesättigten, monocyclischen, heteroaliphatischen Ring darstellen, der gegebenenfalls durch ein Sauerstoffatom oder durch die >N-$CH_3$-Gruppe unterbrochen sein kann

und

Z eine Einfachbindung, ein Sauerstoffatom, die Methylenoder I,2-Ethylengruppe ist.

Die Verbindungen der allgemeinen Formel I können nach Umsetzung mit anorganischen oder organischen Säuren auch in Form ihrer physiologisch verträglichen Salze vorliegen. Als Säuren haben sich beispielsweise Salzsäure, Bromwasserstoffsäure, Schwefelsäure, Methylschwefelsäure, Phosphorsäure, Weinsäure, Fumarsäure, Zitronensäure, Maleinsäure, Bernsteinsäure, Gluconsäure, Äpfelsäure, p-Toluolsulfonsäure, Methansulfonsäure, Amidosulfonsäure oder Cyclohexansulfaminsäure als geeignet erwiesen.

Zur Erläuterung des Erfindungsgegenstandes seien als Beispiele folgende Verbindungen genannt:

II-[4-[2-[(Diethylamino)methyl]-I-piperidinyl]-I-oxobutyl]-5,II-dihydro-6H-pyrido[2,3-b][I,4]benzodiazepin-6-on;

D,L-5,II-Dihydro-II-[4-[2-[(dimethylamino)methyl]-I-piperidinyl]-I-oxobutyl]- 6H-pyrido[2,3-b][I,4]benzodiazepin-6-on;

5,II-Dihydro-II-[4-[2-[(ethylmethylamino)methyl]-I-piperidinyl]-I-oxobutyl]- 6H-pyrido[2,3-b][I,4]benzodiazepin-6-on;

5,II-Dihydro-II-[4-[2-[(dipropylamino)methyl]-I-piperidinyl]-I-oxobutyl]-6H-pyrido[2,3-b][I,4]benzodiazepin-6-on;

II-[4-[2-[(Diethylamino)methyl]-I-pyrrolidinyl]-I-oxobutyl]-5,II-dihydro-6H-pyrido[2,3-b][I,4]benzodiazepin-6-on;

II-[4-[2-[(Diethylamino)methyl]-I-azetidinyl]-I-oxobutyl]-5,II-dihydro-6H-pyrido[2,3-b][I,4]benzodiazepin-6-on;

D,L-II-[4-[2-[2-(Diethylamino)ethyl]-I-piperidinyl]-I-oxobutyl]-5,II-dihydro- 6H-pyrido[2,3-b][I,4]benzodia-

zepin-6-on;

D,L-5,ll-Dihydro-ll-[4-[2-[2-(4-morpholinyl)ethyl]-l-piperidinyl]-l-oxobutyl]- 6H-pyrido[2,3-b][l,4]benzodiazepin-6-on;

5,ll-Dihydro-ll-[4-[2-[2-(hexahydro-lH-l-azepinyl)ethyl]-l-piperidinyl]-l-oxobutyl]-6H-pyrido[2,3-b][l,4]benzodiazepin-6-on;

5,ll-Dihydro-ll-[4-[2-[2-(dimethylamino)ethyl]-l-piperidinyl]-l-oxobutyl]-6H-pyrido[2,3-b][l,4]benzodiazepin-6-on;

D,L-ll-[4-[3-[(Diethylamino)methyl]-4-morpholinyl]-l-oxobutyl]-5,ll-dihydro- 6H-pyrido[2,3-b][l,4]benzodiazepin-6-on;

D-ll-[4-[3-[(Diethylamino)methyl]-4-morpholinyl]-l-oxobutyl]-5,ll-dihydro-6H-pyrido[2,3-b][l,4]benzodiazepin-6-on;

L-ll-[4-[3-[(Diethylamino)methyl]-4-morpholinyl]-l-oxobutyl]-5,ll-dihydro-6H-pyrido[2,3-b][l,4]benzodiazepin-6-on;

ll-[4-[2-[(Diethylamino)methyl]-l-piperidinyl]-l-oxobutyl]-5,ll-dihydro-9-methyl-6H-pyrido[2,3-b][l,4]benzodiazepin-6-on;

ll-[4-[2-[(Diethylamino)methyl]-l-piperidinyl]-l-oxobutyl]-5,ll-dihydro-8-methyl-6H-pyrido[2,3-b][l,4]benzodiazepin-6-on;

ll-[4-[3-[(Diethylamino)methyl]-4-morpholinyl]-l-oxobutyl]-5,ll-dihydro-8-ethyl- 6H-pyrido[2,3-b][l,4]benzodiazepin-6-on;

9-Chlor-ll-[4-[3-[(diethylamino)methyl]-4-morpholinyl]-l-oxobutyl]-5,ll-dihydro- 6H-pyrido[2,3-b][l,4]benzodiazepin-6-on;

8-Brom-ll-[4-[2-[(diethylamino)methyl]-l-piperidinyl]-l-oxobutyl]-5,ll-dihydro- 6H-pyrido[2,3-b][l,4]benzodiazepin-6-on-methansulfonat;

ll-[4-[2-[(l-Azetidinyl)methyl]-l-piperidinyl]-l-oxobutyl]-5,ll-dihydro-6H-pyrido[2,3-b][l,4]benzodiazepin-6-on;

ll-[4-[4-[3-(Diethylamino)propyl]-l-piperidinyl]-l-oxobutyl]-5,ll-dihydro-6H-pyrido[2,3-b][l,4]benzodiazepin-6-on;

5,ll-Dihydro-ll-[4-[2-[(l-pyrrolidinyl)methyl]-l-piperidinyl]-l-oxobutyl]-6H-pyrido[2,3-b][l,4]benzodiazepin-6-on;

5,ll-Dihydro-ll-[4-[4-[4-(l-pyrrolidinyl)butyl]-l-piperidinyl]-l-oxobutyl]-6H-pyrido[2,3-b][l,4]benzodiazepin-6-on;

ll-[4-[3-[4-(Diethylamino)butyl]-l-piperidinyl]-l-oxobutyl]-5,ll-dihydro-6H-pyrido[2,3-b][l,4]benzodiazepin-6-on;

5,ll-Dihydro-ll-[4-[3-[2-(l-pyrrolidinyl)ethyl]-l-piperidinyl]-l-oxobutyl]-6H-pyrido[2,3-b][l,4]benzodiazepin-6-on;

5,ll-Dihydro-ll-[4-[3-[4-(l-pyrrolidinyl)butyl]-l-piperidinyl]-l-oxobutyl]-6H-pyrido[2,3-b][l,4]benzodiazepin-6-on;

ll-[4-[3-[2-(Diethylamino)ethyl]-hexahydro-lH-l-azepinyl]-l-oxobutyl]-5,ll-dihydro-6H-pyrido[2,3-b][l,4]benzodiazepin-6-on;

ll-[4-[2-[(Diethylamino)methyl]-l-piperidinyl]-l-oxobutyl]-6,ll-dihydro-5H-pyrido[2,3-b][l,5]benzodiazepin-5-on;

ll-[4-[2-[2-(Diethylamino)ethyl]-l-piperidinyl]-l-oxobutyl]-6,ll-dihydro-5H-pyrido[2,3-b][l,5]benzodiazepin-5-on;

ll-[4-[3-[(Diethylamino)methyl]-4-morpholinyl]-l-oxobutyl]-6,ll-dihydro-5H-pyrido[2,3-b][l,5]benzodiazepin-5-on;

ll-[4-[4-[3-(Diethylamino)propyl]-l-piperidinyl]-l-oxobutyl]-6,ll-dihydro-5H-pyrido[2,3-b][l,5]benzodiazepin-5-on;

ll-[4-[3-[4-(Diethylamino)butyl]-l-piperidinyl]-l-oxobutyl]-6,ll-dihydro-5H-pyrido[2,3-b][l,5]benzodiazepin-5-on;

5-[4-[3-[2-(Diethylamino)ethyl]-hexahydro-lH-l-azepinyl]-l-oxobutyl]-5,l0-dihydro-llH-dibenzo[b,e][l,4]diazepin-ll-on;

5-[4-[2-[(Diethylamino)methyl]-l-piperidinyl]-l-oxobutyl]-5,l0-dihydro-llH-dibenzo[b,e][l,4]diazepin-ll-on;

5-[4-[2-[2-(Diethylamino)ethyl]-l-piperidinyl]-l-oxobutyl]-5,l0-dihydro-llH-dibenzo[b,e][l,4]diazepin-ll-on;

5-[4-[3-[(Diethylamino)methyl]-4-morpholinyl]-l-oxobutyl]-5,l0-dihydro-llH-dibenzo[b,e][l,4]diazepin-ll-on;

5-[4-[4-[3-(Diethylamino)propyl]-l-piperidinyl]-l-oxobutyl]-5,l0-dihydro-llH-dibenzo[b,e][l,4]diazepin-ll-on;

5-[4-[3-[4-(Diethylamino)butyl]-l-piperidinyl]-l-oxobutyl]-5,l0-dihydro-llH-dibenzo[b,e][l,4]diazepin-ll-on;

4,9-Dihydro-3-methyl-4-[4-[3-[2-(l-piperidinyl)ethyl]-hexahydro-lH-l-azepinyl]- -oxobutyl]-l0H-thieno[3,4-b][l,5]benzodiazepin-l0-on;

4,9-Dihydro-3-methyl-4-[4-[2-[(l-piperidinyl)methyl]-l-piperidinyl]-l-oxobutyl]- l0H-thieno[3,4-b][l,5]benzodiazepin-l0-on;

4,9-Dihydro-3-methyl-4-[4-[2-[2-(l-piperidinyl)ethyl]-l-piperidinyl]-l-oxobutyl]l0H-thieno[3,4-b][l,5]benzodiazepin-l0-on;

4,9-Dihydro-3-methyl-4-[4-[3-[(l-piperidinyl)methyl]-4-morpholinyl]-l-oxobutyl]- l0H-thieno[3,4-b][l,5]benzodiazepin-l0-on;

3-Chlor-4,9-dihydro-4-[4-[3-[(l-piperidinyl)methyl]-4-morpholinyl]-l-oxobutyl]- l0H-thieno[3,4-b][l,5]benzodiazepin-l0-on;

4,9-Dihydro-4-[4-[3-[(diethylamino)methyl]-4-morpholinyl]-l-oxobutyl]-l,3-dimethyl-l0H-thieno[3,4-b][l,5] benzodiazepin-l0-on;

4,9-Dihydro-3-methyl-4-[4-[4-[3-(l-piperidinyl)propyl]-l-piperidinyl]-l-oxobutyl]-l0H-thieno[3,4-b][l,5]benzodiazepin-l0-on;

4,9-Dihydro-3-methyl-4-[4-[3-[(4-(l-piperidinyl)butyl]-l-piperidinyl]-l-oxobutyl]-l0H-thieno[3,4-b][l,5]benzodiazepin-l0-on;

l,3-Dimethyl-4-[4-[3-[(l-piperidinyl)methyl]-4-morpholinyl]-l-oxobutyl]-l,4,9,l0-tetrahydropyrrolo[3,2-b][l,5] benzodiazepin-l0-on;

3-Chlor-l-methyl-4-[4-[3-[(l-piperidinyl)methyl]-4-morpholinyl]-l-oxobutyl]-l,4,9,l0-tetrahydropyrrolo[3,2-b] [l,5]benzodiazepin-l0-on;

l-Methyl-4-[4-[2-[2-(l-piperidinyl)ethyl]-4-morpholinyl]-l-oxobutyl]-l,4,9,l0-tetrahydropyrrolo[3,2-b][l,5]benzodiazepin-l0-on;

l-Methyl-4-[4-[3-[4-(l-piperidinyl)butyl]-l-piperidinyl]-l-oxobutyl]-l,4,9,l0-tetrahydropyrrolo[3,2-b][l,5]benzodiazepin-l0-on;

l,3-Dimethyl-4-[4-[3-[(l-piperidinyl)methyl]-4-morpholinyl]-l-oxobutyl]-l,4,9,l0-tetrahydropyrazolo[4,3-b][l,5] benzodiazepin-l0-on;

l-Methyl-4-[4-[3-[(l-piperidinyl)methyl]-4-morpholinyl]-l-oxobutyl]-l,4,9,l0-tetrahydropyrazolo[4,3-b][l,5]benzodiazepin-l0-on;

l,3-Dimethyl-4-[4-[3-[4-(l-piperidinyl)butyl]-l-piperidinyl]-l-oxobutyl]-l,4,9,l0-tetrahydropyrazolo[4,3-b][l,5] benzodiazepin-l0-on;

l-Methyl-4-[4-[2-[2-(l-piperidinyl)ethyl]-4-morpholinyl]-l-oxobutyl]-l,4,9,l0-tetrahydropyrazolo[4,3-b][l,5]benzodiazepin-l0-on;

Erfindungsgemäß erhält man die neuen basisch substituierten kondensierten Diazepinone der allgemeinen Formel I nach folgenden Verfahren:

a) Basisch substituierte kondensierte Diazepinone der allgemeinen Formel Ia,

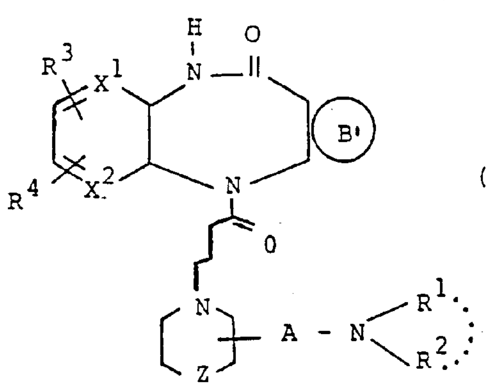

(Ia)

in der

$R^l$, $R^2$, $R^3$, $R^4$, $X^l$, $X^2$, A und Z die oben angegebenen Bedeutungen haben und 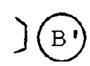 einen der zweiwertigen Reste (S), (U), (V), (W) oder (T′)

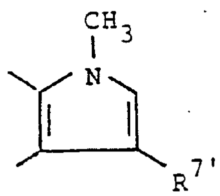

(T')

darstellt, wobei $R^{7'}$ ein Chloratom oder eine Methylgruppe ist,

erhält man durch Umsetzung von Halogenacylverbindungen der allgemeinen Formel II,

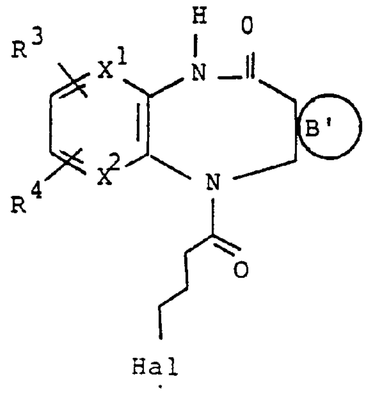

(II)

in der

$R^3$, $R^4$, 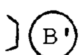 , $X^1$ und $X^2$ die angegebenen Bedeutungen haben und

Hal ein Chlor-, Brom- oder Iodatom ist, mit sekundären Aminen der allgemeinen Formel III,

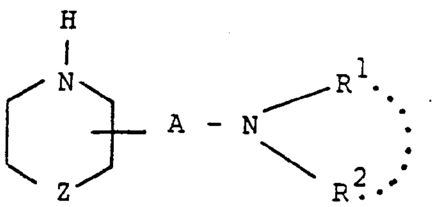

(III)

in der

$R^1$, $R^2$, A und Z die eingangs definierten Bedeutungen besitzen.

Die Aminierung erfolgt in einem inerten Lösungsmittel bei Temperaturen zwischen -10°C und der Siedetemperatur des Lösungsmittels, vorzugsweise entweder mit wenigstens 2 Molen sekundärem Amin der allgemeinen Formel III oder mit 1 bis 2 Molen eines sekundären Amins der allgemeinen Formel III und einer Hilfsbase. Als Lösungsmittel kommen beispielsweise chlorierte Kohlenwasserstoffe, wie Methylenchlorid, Chloroform oder Dichlorethan; offenkettige oder cyclische Ether, wie Diethylether, Tetrahydrofuran oder Dioxan; aromatische Kohlenwasserstoffe, wie Benzol, Toluol, Xylol, Chlorbenzol oder Pyridin; Alkohole, wie Ethanol oder Isopropanol; Ketone, wie Aceton; Acetonitril, Dimethylformamid oder 1,3-Dimethyl-2-imidazolidinon in Frage. Als Hilfsbasen seien beispielsweise tertiäre organische Basen, wie Triethylamin, N-Methylpiperidin, Diethylanilin, Pyridin und 4-(Dimethylamino)pyridin oder anorganische Basen, wie Alkalimetall- oder Erdalkalimetallcarbonate oder -hydrogencarbonate, -hydroxide oder -oxide genannt. Gegebenenfalls kann die Reaktion durch Zusatz von Alkalimetalliodiden beschleunigt werden. Die Reaktionszeiten betragen je nach Menge und Art des eingesetzten Amins der allgemeinen Formel III zwischen 15 Minuten und 80 Stunden.

b) Basisch substituierte kondensierte Diazepinone der allgemeinen Formel Ia lassen sich auch durch Umsetzung von Tricyclen der allgemeinen Formel IV,

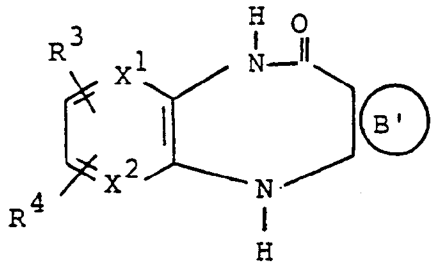

(IV)

in der
die Reste $R^3$, $R^4$, $X^1$, $X^2$ und 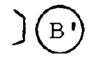 wie oben definiert sind, mit Carbonsäurederivaten der allgemeinen Formel V,

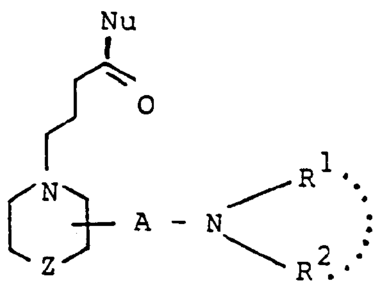

(V)

worin
$R^1$, $R^2$, A und Z die oben angegebenen Bedeutungen haben und Nu eine nucleofuge Gruppe bzw. Abgangsgruppe darstellt, herstellen.

Die Umsetzung der Verbindungen der allgemeinen Formel IV mit den Säurederivaten der allgemeinen Formel V erfolgt in an sich bekannter Weise. Die Abgangsgruppe Nu ist eine Gruppe, die gemeinsam mit der Carbonylgruppe, an die sie gebunden ist, ein reaktives Carbonsäurederivat bildet. Als reaktive Carbonsäurederivate seien beispielsweise Säurehalogenide, -ester, -anhydride oder gemischte -anhydride, wie sie aus Salzen der entsprechenden Säuren (Nu=OH) und Säurechloriden, wie Phosphoroxidchlorid, Diphosphorsäuretetrachlorid oder Chlorameisensäureestern gebildet werden oder die bei der Umsetzung von Verbindungen der allgemeinen Formel V (Nu=OH) mit N-Alkyl-2-halogenpyridiniumsalzen entstehenden N-Alkyl-2-acyloxypyridiniumsalze genannt.

Bevorzugt wird die Reaktion mit den gemischten Anhydriden starker Mineralsäuren, insbesondere der Dichlorphosphorsäure, durchgeführt. Die Reaktion wird gegebenenfalls in Gegenwart eines säuebindenden Mittels (Protonenakzeptors) durchgeführt. Als geeignete Protonenakzeptoren seien beispielsweise Alkalimetallcarbonate oder -hydrogencarbonate, wie Natriumcarbonat oder Kaliumhydrogencarbonat; tertiäre organische Amine, wie Pyridin, Triethylamin, Ethyldiisopropylamin, 4-(Dimethylamino)pyridin, oder Natriumhydrid genannt.

Die Reaktion wird bei Temperaturen zwischen -25°C und 130°C in einem inerten Lösungsmittel durchgeführt. Als inerte Lösungsmittel kommen beispielsweise chlorierte aliphatische Kohlenwasserstoffe, wie Methylenchlorid, 1,2-Dichlorethan; offenkettige oder cyclische Ether, wie Diethylether, Tetrahydrofuran oder 1,4-Dioxan; aromatische Kohlenwasserstoffe, wie Benzol, Toluol, Xylol, o-Dichlorbenzol; polare aprotische Lösungsmittel wie Acetonitril, Dimethylformamid, 1,3-Dimethyl-2-imidazolidinon oder Hexamethylphosphorsäuretriamid; oder Gemische davon in Frage. Die Reaktionszeiten betragen je nach Menge und Art des eingesetzten Acylierungsmittels der allgemeinen Formel V zwischen 15 Minuten und 80 Stunden. Es ist nicht nötig, die Verbindungen der allgemeinen Formel V in reiner Form herzustellen, sie können vielmehr im Reaktionsansatz in bekannter Weise in situ erzeugt werden.

c.) Die unter die allgemeine Formel I fallenden neuen Pyrrolo-kondensierten Diazepinone der allgemeinen Formel Ib,

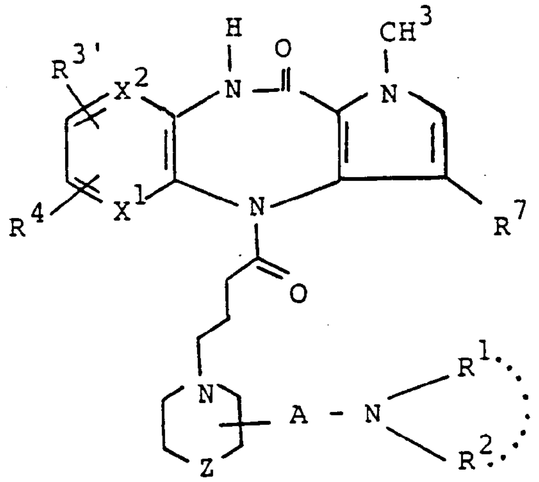

(Ib)

worin

$R^I$, $R^2$, $R^4$, $X^I$, $X^2$ und A wie eingangs definiert sind, Z die eingangs genannten Bedeutungen it Ausnahme eines Schwefelatoms hat,

$R^{3'}$ eine Alkylgruppe mit I bis 4 C-Atomen oder ein Wasserstoffatom und

$R^7$ ein Wasserstoffatom darstellen, können auch durch Hydrogenolyse aus solchen Verbindungen der allgemeinen Formel Ib hergestellt werden, in denen $R^7$ ein Chloratom bedeutet.

Die Hydrogenolyse wird in Gegenwart von Katalysatoren von Metallen aus der VIII. Nebengruppe des Periodensystems der Elemente, beispielsweise von Palladium auf Tierkohle, Palladium auf Bariumsulfat, Raney-Nickel oder Raney-Cobalt, und bei Wasserstoffdrucken von I bis 300 bar und Temperaturen von 0°C bis I30°C in Gegenwart von Lösungsmitteln, beispielsweise Alkoholen, wie Methanol, Ethanol; Ethern wie Dioxan, Tetrahydrofuran; Carbonsäuren, beispielsweise Essigsäure; oder tertiären Aminen, beispielsweise Triethylamin, durchgeführt. Arbeitet man dabei in Abwesenheit zusätzlicher Chlorwasserstoff-Akzeptoren, beispielsweise von Natriumcarbonat, Kaliumhydrogencarbonat, Triethylamin oder Natriumacetat, so entstehen direkt die Hydrochloride der gesuchten Verbindungen, die nach Entfernung des Katalysators durch Eindampfen der Reaktionslösung isoliert werden können. Ersetzt man in der vorstehenden Hydrogenolyse-Reaktion den Wasserstoff durch Ameisensäure, so gelingt die Umsetzung prinzipiell schon bei drucklosem Arbeiten. Bei dieser Variante haben sich besonders die Umsetzung mit Ameisensäure in Gegenwart von Dimethylformamid als Lösungsmittel und von Palladium auf Kohle als Katalysator bei Temperaturen zwischen 70 und II0°C, sowie die Reduktion mit Triethylammoniumformiat in Gegenwart überschüssigen Triethylamins und von Palladium auf Tierkohle oder von Palladiumacetat und Triarylphosphinen, wie Triphenylphosphin, Tris (o-tolyl)phosphin, Tris-(2,5-diisopropylphenyl)phosphin, bei Temperaturen zwischen 40 und II0°C, bewährt.

So erhaltene Basen der allgemeinen Formel I können anschließend in ihre Säureadditionssalze oder erhaltene Säureadditionssalze in die freien Basen oder andere pharmakologisch verträgliche Säureadditionssalze übergeführt werden.

Die erfindungsgemäßen basisch substituierten kondensierten Diazepinone der allgemeinen Formel I enthalten bis zu zwei unabhängige chirale Elemente, davon ein asymmetrisches Kohlenstoffatom in der Seitenkette. Als zweites chirales Element ist der acylierte Tricyclus selbst anz sehen, der in zwei spiegelbildlichen Formen vorliegen kann. Von der Natur des Tricyclus hängt es ab, ob die Energiebarriere für eine Inversion an diesem Zentrum so hoch ist, daß die einzelnen Isomeren bei Zimmertemperatur stabil sind und isolierbar werden. Es hat sich gezeigt, daß solche Verbindungen der allgemeinen Formel I, in der $X^2$ die CH-Gruppe ist und 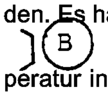 den o-Phenylenrest bedeutet, stets in zwei diastereomeren Formen vorliegen, die bei Zimmertemperatur in die Komponenten getrennt werden können. Die einzelnen Diastereomeren sind in kristallinem Zustand völlig stabil, gehen in Lösung und bei Zimmertemperatur aber mit einer Halbwertzeit von einigen Tage wieder ins ursprüngliche Gemisch über, sofern raumerfüllende peri-ständige Substituenten, wie Chlor oder Methyl, fehlen.

Bei Verbindungen der allgemeinen Formel I, in der $X^2$ das N-Atom und 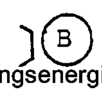 die in den peri-Stellungen unsubstituierte o-Phenylengruppe bedeutet, ist die erforderliche Aktivierungsenergie so stark vermindert, daß Diastereomere bei Zimmertemperatur - außer durch die komplexen $^IH$-NMR-Spektren - nicht mehr nachgewiesen geschweige denn präparativ getrennt werden können.

Die erfindungsgemäßen aminoacylierten kondensierten Diazepinone der allgemeinen Formel I enthalten als in der Regel zwei chirale Zentren, von denen eines bei Zimmertemperatur unter Umständen nicht konfigurationsstabil ist. Diese Verbindungen können deshalb in zwei diastereomeren Formen auftreten, die ihrerseits jeweils in enantiomere (+)- und (-)-Formen getrennt werden können. Die Erfindung umfaßt die einzelnen Isomeren ebenso wie ihre Gemische. Die Trennung der jeweiligen Diastereomeren gelingt auf Grund der unterschiedlichen physiko-chemischen Eigenschafte, z. B. durch fraktioniertes Umkristallisieren aus geeigneten Lösungsmitteln, durch Hochdruckflüssigkeitschromatographie, Säulenchromatographie oder gaschromatographische Verfahren.

Die Spaltung evtl. Racemate der Verbindungen der allgemeinen Formel I kann nach bekannten Verfahren durchgeführt werden, beispielsweise unter Verwendung einer optisch aktiven Säure, wie (+)- oder (-)-Weinsäure, oder eines Derivats davon, wie (+)- oder (-)-Diacetylweinsäure, (+)- oder (-)-Monomethyltartrat oder (+)-Camphersulfonsäure.

Nach einem üblichen Verfahren zur Isomerentrennung wird das Racemat einer Verbindung der allgemeinen Formel I mit einer der vorstehend angegebenen optisch aktivensäuren in äquimolarer Menge in einem Lösungsmittel umgesetzt und die erhaltenen kristallinen diastereomeren Salze werden unter Ausnutzung ihrer verschiedenen Löslichkeit getrennt. Diese Umsetzung kann in jeder Art von Lösungsmittel durchgeführt werden, solange dieses einen ausreichenden Unterschied in der Löslichkeit der Salze aufweist. Vorzugsweise werden Methanol, Ethanol oder deren Gemische, beispielsweise im Volumenverhältnis 50:50, verwendet. Sodann wird jedes der diastereomeren Salze in Wasser gelöst, mit einer Base, wie Natriumhydroxid oder Kaliumhydroxid, neutralisiert und dadurch die entsprechende freie Verbindung in cer (+)- oder (-)-Form erhalten.

Jeweils nur ein Enantiomeres bzw. ein Gemisch zweier optisch aktiver unter die allgemeine Formel I fallender diastereomerer Verbindungen wird auch dadurch erhalten, daß man die oben beschriebenen Synthesen mit nur einem Enantiomeren der allgemeinen Formeln III bzw. V durchführt.

Die zur Synthese der kondensierten Diazepinone der allgemeinen Formel I erforderlichen Ausgangsmaterialien der Formeln II, III, IV und V sind bekannt oder werden in Analogie zu beschriebenen Verfahren hergestellt; siehe z. B die Angaben in den Patentschriften US-Patent 4 550 l07, EP-A-0 254 955 und DE-A-36 43 666.

Die basisch substituierten kondensierten Diazepinone der allgemeinen Formel I und ihre Säureadditionssalze besitzen wertvolle Eigenschaften; insbesondere besitzen sie bei ausgeprägter Hydrolysestabilität, hoher Selektivität und guter Resorption nach oraler Gabe günstige Effekte auf die Herzfrequenz und sind angesichts fehlender magensäuresekretionshemmender, salivationshemmender und mydriatischer Einflüsse als vagale Schrittmacher zur Behandlung von Bradycardien und Bradyarrhythmien in der Human- und auch der Veterinärmedizin geeignet; ein Teil der Verbindungen zeigt auch spasmolytische Eigenschaften auf periphere Organe, insbesondere Colon und Blase.

Eine günstige Relation zwischen tachycarden Wirkungen einerseits und den bei Therapeutika mit anticholinerger Wirkkomponente auftretenden unerwünschten Wirkungen auf die Pupillenweite und Tränen-, Speichel- und Magensäuresekretion andererseits ist für die therapeutische Verwendung der Substanzen von besonderer Wichtigkeit. Die folgenden Versuche zeigen, daß die erfindungsgemäßen Verbindungen diesbezüglich überraschend günstige Relationen aufweisen.

Bindungsstudien an muscarinischen Rezeptoren:

<u>Bestimmung des $IC_{50}$-Wertes in vitro</u>

Als Organspender dienten männliche Sprague-Dawley-Ratten mit l80-220 g Körpergewicht. Nach Entnahme von Herz, Submandibularis und Großhirnrinde wurden alle weiteren Schritte in eiskaltem Hepes-HCl-Puffer (pH 7,4; 100 m molar NaCl, 10 m molar $MgCl_2$) vorgenommen. Das Gesamtherz wurde mit einer Schere zerkleinert. Alle Organe wurden abschließend in einem Potter homogenisiert.

Für den Bindungstest wurden die Organhomogenate in folgender Weise verdünnt:

| | |
|---|---|
| Gesamtherz | 1: 400 |
| Großhirnrinde | 1:3000 |
| Submandibularis | 1: 400 |

Die Inkubation der Organhomogenate erfolgte bei einer bestimmten Konzentration des Radioliganden und einer Konzentrationsreihe der nichtradioaktiven Testsubstanzen im Eppendorf-Zentrifugenröhrchen bei 30°C. Die Inkubationsdauer betrug 45 Minuten. Als Radioligand wurde 0,3 n molar $^3$H-N-Methylscopolamin ($^3$H-NMS) verwendet. Die Inkubation wurde durch Zugabe von eiskaltem Puffer mit nachfolgender Vakuumfiltration beendet. Die Filter wurden mit kaltem Puffer gespült und ihre Radioaktivität bestimmt. Sie repräsentiert die Summe von spezifischer und unspezifischer Bindung von $^3$H-NMS. Der Anteil der unspezifischen Bindung wurde definiert als jene Radioaktivität, die in Anwesenheit von l μ molar Chinuclidinylbenzilat gebunden wurde. Es wurden immer Vierfachbestimmungen vorgenommen. Die $IC_{50}$-Werte der nicht-markierten Testsubstanzen wurden graphisch bestimmt. Sie repräsentieren jene Konzentration der Testsubstanz, bei welcher die spezifische Bindung von $^3$H-NMS an die muscarinischen Rezeptoren in den verschiedenen Organen um 50 % gehemmt wurde. Die Ergebnisse sind aus der Tabelle I zu ersehen.

B. Untersuchung auf funktionelle Selektivität der antimuscarinischen Wirkung

Substanzen mit antimuscarinischen Eigenschaften inhibieren die Wirkungen von exogen zugeführten Agonisten oder von Acetylcholin, das aus cholinergen Nervenendigungen freigesetzt wird. Im folgenden wird eine Beschreibung von Methoden wiedergegeben, die zur Erfassung von cardioselektiven Antimuscarinica geeignet sind.

"In vivo" Methoden

Die angewandten Methoden hatten zum Ziel, die Selektivität der antimuscarinischen Wirkung zu bestätigen. Jene Substanzen, die auf der Basis von "in vitro" Untersuchungen ausgewählt worden waren, wurden auf ihre

l. $M_l$-/$M_2$-Selektivität an der Ratte

2. Speichelsekretionshemmende Wirkung an der Ratte und

3. Hemmung der Acetylcholinwirkung auf Blase, Bronchien und Herzfrequenz des Meerschweinchens

untersucht.

l. $M_l$-/$M_2$-Selektivität an der Ratte

Die angewandte Methode wurde von Hammer und Giachetti (Life Sciences 3l, 299l-2998 (l982)) beschrieben. 5 Minuten nach intravenöser Injektion steigender Dosen der Substanz wurden entweder der rechte Vagus elektrisch stimuliert (Frequenz: 25 Hz; Pulsbreite: 2ms; Reizdauer: 30s; Voltzahl: supramaximal) oder 0,3 mg/kg McN-A-343 in männliche THOM-Ratten intravenös injiziert. Die durch Vagusstimulation hervorgerufene Bradykardie und der durch McN-A-343 verursachte Blutdruckanstieg wurden bestimmt. Die Dosis der Substanzen, die entweder die vagale Bradykardie ($M_2$) oder den Blutdruckanstieg ($M_l$) um 50% verminderte, wurde graphisch ermittelt. Ergebnisse siehe Tabelle II.

2. Speichelsekretionshemmende Wirkung an der Ratte

Nach Lavy und Mulder (Arch. int. Pharmacodyn. l78, 437-445, (l969)) erhielten mit l,2 g/kg Urethan narkotisierte männliche THOM-Ratten steigende Dosen der Substanz i.v.. Die Speichelsekretion wurde durch s.c. Gabe von 2 mg/kg Pilocarpin ausgelöst. Der Speichel wurde mit Fließpapier aufgesaugt, die von ihm eingenommene Fläche alle 5 Minuten planimetrisch bestimmt. Die Dosis der Substanz, die das Speichelvolumen um 50% verminderte, wurde graphisch ermittelt. Ergebnisse siehe Tabelle II.

3. Hemmung der Acetylcholinwirkung auf Blase, Bronchien und Herzfrequenz des Meerschweinchens

Am narkotisierten Meerschweinchen wurden 5 Minuten nach Gabe der Prüfsubstanz l0 μg/kg Acetylcholin sowohl intravenös als auch gleichzeitig intraarteriell injiziert. Dabei wurde die Herzfrequenz durch extrakorporale Ableitung des EKG, der Ausatemwiderstand nach Konzett-Rößler und die Kontraktion der freigelegten Harnblase direkt registriert. Für die Hemmung der Acetylcholinwirkung an den untersuchten Organen wurden Dosis-Wirkungskurven aufgenommen und daraus -log $ED_{50}$-Werte bestimmt. Ergebnisse siehe Tabelle III.

Nach den vorstehenden Angaben wurden beispielsweise die folgenden Verbindungen untersucht:

A = ll-[4-[3-[(Diethylamino)methyl]-4-morpholinyl]-l-oxobutyl]-5,ll-dihydro-6H-pyrido[2,3- b][l,4]benzodiazepin-6-on;

B = ll-[4-[2-[(Diethylamino)methyl]-l-piperidinyl]-l-oxobutyl]-5,ll-dihydro-9-methyl- 6H-pyrido[2,3-b][l,4]benzodiazepin-6-on

C = 5, ll-Dihydro-ll-[4-[2-[(l-pyrrolidinyl)methyl]-l-piperidinyl]-l-oxobutyl]-6H-pyrido[2,3- b][l,4]benzodiazepin-6-on

und als Vergleichssubstanzen

D = ll-[[2-[(Diethylamino)methyl]-l-piperidinyl]acetyl]-5,ll-dihydro-6H-pyrido[2,3- b][l,4]benzodiazepin-6-on (siehe US-Patent Nr. 4 550 l07),
E = 5,ll-Dihydro-ll-[(4-methyl-l-piperazinyl)acetyl]-6H-pyrido[2,3-b][l,4]benzodiazepin-6-on (siehe US-Patent Nr. 3 660 380) und
F = Atropin

Tabelle I:

Rezeptor-Bindungs-Tests, in vitro:

Ergebnisse:

| Substanz | Rezeptor-Bindungs-Tests $IC_{50}$ [$nMl^{-1}$] Cortex (C) | Herz (H) | Submandibularis (SM) |
|---|---|---|---|
| A | 100 | 40 | 100 |
| B | 30 | 6 | 60 |
| C | 20 | 4 | 40 |
| D | 1200 | 140 | 5000 |
| E | 100 | 1500 | 200 |
| F | 2 | 4 | 4 |

Die Angaben in der vorstehenden Tabelle I beweisen, daß die neuen Verbindungen der allgemeinen Formel I zwischen muscarinischen Rezeptoren verschiedener Gewebe unterscheiden. Dies folgt aus den beträchtlich niedrigeren $IC_{50}$-Werten bei Untersuchung an Präparaten aus dem Herzen gegebenüber solchen aus der Großhirnrinde.

Tabelle II:

$M_1/M_2$-Selektivität und Speichelsekretionshemmung an der Ratte:

Ergebnisse:

| | -log $ED_{50}$ [$Mkg^{-1}$] | | |
|---|---|---|---|
| Substanz | Herz | Blutdruck | Salivationshemmung |
| A | 7,29 | 6,26 | 5,96 |
| B | 7,21 | 6,30 | 5,95 |
| C | 7,33 | 6,66 | 5,78 |
| D | 6,42 | 5,63 | 5,00 |
| E | 5,60 | 6,94 | 6,22 |
| F | 7,94 | 7,34 | 7,60 |

Tabelle III:

Hemmung der Acetylcholinwirkung auf Blase, Bronchien und Herzfrequenz des Meerschweinchens:

Ergebnisse:

| | $-\log ED_{50}$ [$Molkg^{-1}$] | | |
|---|---|---|---|
| Substanz | Herz | Bronchien | Blase |
| A | 7,55 | 7,15 | 6,00 |
| B | 7,15 | 6,82 | 6,12 |
| C | 7,56 | 7,29 | 6,50 |
| D | 5,84 | 5,58 | 4,73 |
| E | 5,85 | 6,57 | 5,36 |
| F | 7,70 | 7,96 | 7,03 |

Aus den pharmakologischen Daten der vorstehenden Tabellen II und III ergibt sich - in völliger Übereinstimmung mit den Rezeptor-Bindungs-Studien -, daß die Herzfrequenz durch die genannten Verbindungen bereits bei Dosierungen gesteigert wird, bei denen noch keine Einschränkung der Speichelsekretion beobachtet wird.

Außerdem deuten die pharmakologischen Daten der vorstehenden Tabelle III auf ein überraschend großes Unterscheidungsvermögen zwischen Herz und glatter Muskulatur.

Die genannten Substanzen weisen gegenüber der bereits bekannten Verbindung D eine wesentlich gesteigerte Wirkungsstärke auf. Dabei bleibt die therapeutisch nutzbare Selektivität erhalten. Dies führt zu einer geringeren Substanzbelastung des Patienten, ohne das Risiko muskarinischer Nebenwirkungen zu erhöhen.

Ferner sind die erfindungsgemäß hergestellten Verbindungen gut verträglich, so konnten selbst bei den höchsten applizierten Dosen bei den pharmakologischen Untersuchungen keine toxischen Nebenwirkungen beobachtet werden.

Zur pharmazeutischen Anwendung lassen sich die Verbindungen der allgemeinen Formel I in an sich bekannter Weise in die üblichen pharmazeutischen Zubereitungsformen, z.B. in Lösungen, Suppositorien, Tabletten, Dragées, Kapseln oder Teezubereitungen einarbeiten. Die Tagesdosis liegt im allgemeinen zwischen 0,02 und 5 mg/kg, vorzugsweise 0,02 und 2,5 mg/kg, insbesondere 0,05 und l,0 mg/kg Körpergewicht, die gegebenenfalls in Form mehrerer, vorzugsweise l bis 3 Einzelgaben, zur Erzielung der gewünschten Ergebnisse verabreicht wird.

Die nachfolgenden Beispiele sollen die Erfindung näher erläutern:

Für alle Verbindungen liegen befriedigende Elementaranalysen, IR-, UV-, $^1H$-NMR-, häufig auch Massenspektren vor.

Beispiel I

II-[4-[2-[(Diethylamino)methyl]-I-piperidinyl]-I-oxobutyl]-5,II-dihydro-6H-pyrido[2,3-b][I,4]benzodiazepin-6-on

Die Mischung aus 9,5 g (0,03 mol) II-(4-Chlor-I-oxobutyl)-5,II-dihydro-6H-pyrido[2,3-b][I,4]benzodiazepin-6-on, I00 ml wasserfreiem Dimethylformamid, 5,6 g (0,033 mol) 2-[(Diethylamino)methyl]piperidin und 0,5 g Natriumiodid wurde 7 Tage lang bei einer Reaktionstemperatur von 50°C gerührt. Man engte im Wasserstrahlvakuum ein, stellte den Rückstand deutlich natronalkalisch und extrahierte erschöpfend mit Dichlormethan. Die Dichlormethanextrakte wurden vereinigt, über Natriumsulfat getrocknet und eingedampft. Das so erhaltene Rohprodukt (I4,0 g) wurde an Kieselgel als stationärer Phase und unter Verwendung von Dichlormethan/Essigsäureethylester/Cyclohexan/Methanol/konz. Ammoniak 58,5/25,I/7,7/7,7/I,0/ (v/v/v/v/v) zum Eluieren säulenchromatographisch gereinigt. Die entsprechenden eingedampften Eluate kristallisierten beim Anreiben mit t-Butyl-methylether. Nach dem Umkristallisieren aus Essigsäureethylester unter Verwendung von Tierkohle erhielt man 3,8 g (28 % der Theorie) an farblosen Kristallen vom Fp. I43-I44°C; $R_F$ 0.7 (Macherey-Nagel, Polygram(R) SIL G/$UV_{254}$, pre-coated plastic sheets for TLC; Fließmittel: Dichlormethan/Cyclohexan/Methanol/konz. Ammoniak 67,6/I5,2/I5,2/2 (v/v/v/v)) bzw. $R_F$ 0,3 (Fließmittel: Dichlormethan/Essigsäureethylester/Cyclohexan/Methanol/konz. Ammoniak 58,5/25,I/7,7/7,7/I,0 (v/v/v/v/v).

| $C_{26}H_{35}N_5O_2$ | | (449,59) | | | | |
|---|---|---|---|---|---|---|
| Ber.: | C | 69,46 | H | 7,85 | N | 15,58 |
| Gef.: | | 69,69 | | 7,80 | | 15,60 |

Beispiel 2

5,II-Dihydro-II-[4-[2-[(dimethylamino)methyl]-I-piperidinyl]-I-oxobutyl]-6H-pyrido[2,3-b][I,4]benzodiazepin-6-on

Hergestellt analog Beispiel I aus II-(4-Chlor-I-oxobutyl)-5,II-dihydro-6H-pyrido[2,3-b][I,4]benzodiazepin-6-on und 2-[(Dimethylamino)methyl]piperidin in einer Ausbeute von 33 % der Theorie. Farblose Kristalle vom Fp. I57-I58°C (Acetonitril).

Beispiel 3

II-[4-[2-[2-(Diethylamino)ethyl]-I-piperidinyl]-I-oxobutyl]-5,II-dihydro-6H-pyrido[2,3-b][I,4]benzodiazepin-6-on

Hergestellt analog Beispiel I aus II-(4-Chlor-I-oxobutyl)-5,II-dihydro-6H-pyrido[2,3-b][I,4]benzodiazepin-6-on und 2-[2-(Diethylamino)ethyl]piperidin in einer Ausbeute von 3I % der Theorie. Farblose Kristalle vom Fp. 86-88°C; $R_F$ 0,55 (Macherey-Nagel, Polygram(R) SIL G/$UV_{254}$, pre-coated plastic sheets for TLC; Fließmittel: Dichlormethan/Cyclohexan/Methanol/konz. Ammoniak 67,6/I5,2/I5,2/2,0 (v/v/v/v).

| $C_{27}H_{37}N_5O_2$ | | (463,62) | | | | |
|---|---|---|---|---|---|---|
| Ber.: | C | 69,95 | H | 8,04 | N | 15,11 |
| Gef.: | | 69,97 | | 7,97 | | 15,00 |

Beispiel 4

5,II-Dihydro-II-[4-[2-[2-(dimethylamino)ethyl]-I-piperidinyl]-I-oxobutyl]-6H-pyrido[2,3-b][I,4]benzodiazepin-6-on

Hergestellt analog Beispiel I aus II-(4-Chlor-I-oxobutyl)-5,II-dihydro-6H-pyrido[2,3-b][I,4]benzodiazepin-6-on und 2-[2-(Dimethylamino)ethyl]piperidin in einer Ausbeute von 6 % der Theorie. Farblose Kristalle vom Fp. 94-96°C (Diisopropylether/Cyclohexan I:I v/v), löslich in Wasser.

Beispiel 5

Il-[4-[3-[(Diethylamino)methyl]-4-morpholinyl]-l-oxobutyl]-5,Il-dihydro-6H-pyrido[2,3-b][l,4]benzodiazepin-6-on

Hergestellt analog Beispiel I aus Il-(4-Chlor-l-oxobutyl)-5,Il-dihydro-6H-pyrido[2,3-b][l,4]benzodiazepin-6-on und 3-[(Diethylamino)methyl]morpholin in einer Ausbeute von 8 % der Theorie. Farblose Kristalle vom Fp. l08-lI0°C (t-Butylmethylether), leicht löslich in Wasser.

$C_{25}H_{33}N_5O_2$ (451,57)

| | | | | | | |
|---|---|---|---|---|---|---|
| Ber.: | C | 66,50 | H | 7,37 | N | 15,51 |
| Gef.: | | 66,65 | | 7,17 | | 15,42 |

Beispiel 6

Il-[4-[2-[(Diethylamino)methyl]-l-piperidinyl]-l-oxobutyl]-5,Il-dihydro-9-methyl-6H-pyrido[2,3-b][l,4]benzodiazepin-6-on

Hergestellt analog Beispiel I aus Il-(4-Chlor-l-oxobutyl)-5,Il-dihydro-9-methyl-6H-pyrido[2,3-b][l,4]benzodiazepin-6-on (Fp. l78°C [Acetonitril]) und 2-[(Diethylamino)methyl]piperidin in einer Ausbeute von 53 % der Theorie. Farblose Kristalle vom Fp. l54-l56°C (Acetonitril).

$C_{27}H_{37}N_5O_2$ (463,62)

| | | | | | | |
|---|---|---|---|---|---|---|
| Ber.: | C | 69,95 | H | 8,04 | N | 15,11 |
| Gef.: | | 70,00 | | 8,14 | | 15,14 |

Beispiel 7

8-Brom-Il-[4-[2-[(diethylamino)methyl]-l-piperidinyl]-l-oxobutyl]-5,Il-dihydro-6H-pyrido[2,3-b][l,4]benzodiazepin-6-on

Hergestellt analog Beispiel I aus 8-Brom-Il-(4-chlor-l-oxobutyl]-5,Il-dihydro-6H-pyrido[2,3-b][l,4]benzodiazepin-6-on und 2-[(Diethylamino)methyl]piperidin in einer Ausbeute von 50 % der Theorie. Farbloses, sprödes, mit Isooctan sandig verreibbares, in Wasser schwer lösliches Harz; $R_F$ 0,3 (Macherey-Nagel, Polygram(R) SIL G/$UV_{254}$, pre-coated plastic sheets for TLC; Fließmittel: Dichlormethan/Essigsäureethylester/Cyclohexan/Methanol/konz. Ammoniak 58/25/8/8/l (v/v/v/v/v).

$C_{26}H_{34}BrN_5O_2$ (528,49)

| | | | | | | | | |
|---|---|---|---|---|---|---|---|---|
| Ber.: | C | 59,09 | H | 6,48 | Br | 15,12 | N | 13,25 |
| Gef.: | | 58,99 | | 6,42 | | 15,30 | | 13,13 |

Beispiel 8

Il-[4-[4-[3-Diethylamino)propyl]-l-piperidinyl]]-l-oxobutyl]-5,Il-dihydro-6H-pyrido[2,3-b][l,4]benzodiazepin-6-on

Hergestellt analog Beispiel I aus Il-(4-Chlor-l-oxobutyl)-5,Il-dihydro-6H-pyrido[2,3-b][l,4]benzodiazepin-6-on und 4-[3-(Diethylamino)propyl]piperidin in einer Ausbeute von 4,2 % der Theorie. Farblose Kristalle vom Fp. l64-l65°C (Essigsäureethylester); $R_F$ 0.4 (Merck, DC-Fertigplatten, Kieselgel 60F-254. Fließmittel: Dichlormethan/Methanol/Cyclohexan/konz. Ammoniak 68/l5/l5/2 v/v/v/v).

Beispiel 9

ll-[4-[3-[3-(Diethylamino)propyl)-l-piperidinyl]-l-oxobutyl]-5,ll-dihydro-6H-pyrido[2,3-b][l,4]benzodiazepin-6-on

Hergestellt analog Beispiel l aus ll-(4-Brom-l-oxobutyl)-5,ll-dihydro-6H-pyrido[2,3-b][l,4]benzodiazepin-6-on (Fp. l75-l80°C) und 3-[3-(Diethylamino)propyl]piperidin in einer Ausbeute von l4 % der Theorie. Farblose Kristalle vom Fp. l03-l05°C (Acetonitril).

| | | | | | | |
|---|---|---|---|---|---|---|
| $C_{28}H_{39}N_5O_2$ | | (477,65) | | | | |
| Ber.: | C | 70,41 | H | 8,23 | N | 14,66 |
| Gef.: | | 70,26 | | 8,16 | | 14,55 |

Beispiel 10

5,ll-Dihydro-ll-[4-[2-[(l-pyrrolidinyl)methyl]-l-piperidinyl]-l-oxobutyl]-6H-pyrido[2,3-b][l,4]benzodiazepin-6-on

Hergestellt analog Beispiel l aus ll-(4-Chlor-l-oxobutyl)-5,ll-dihydro-6H-pyrido[2,3-b][l,4]benzodiazepin-6-on und 2-[(l-Pyrrolidinyl)methyl]piperidin in einer Ausbeute von 4,7 % der Theorie. Farblose Kristalle vom Fp. l44-l45°C (Acetonitril).

| | | | | | | |
|---|---|---|---|---|---|---|
| $C_{26}H_{33}N_5O_2$ | | (447,60) | | | | |
| Ber.: | C | 69,77 | H | 7,43 | N | 15,65 |
| Gef.: | | 69,93 | | 7,43 | | 15,77 |

Beispiel 11

5,ll-Dihydro-ll-[4-[4-[4-(l-pyrrolidinyl)butyl]-l-piperidinyl]-l-oxobutyl]- 6H-pyrido[2,3-b][l,4]benzodiazepin-6-on

Hergestellt analog Beispiel l aus ll-(4-Chlor-l-oxobutyl)-5,ll-dihydro-6H-pyrido[2,3-b][l,4]benzodiazepin-6-on und 4-[4-(l-Pyrrolidinyl)butyl]piperidin in einer Ausbeute von 36 % der Theorie. Farblose Kristalle vom Fp. l52-l54°C (aus Acetonitril unter Verwendung von Aktivkohle).

| | | | | | | |
|---|---|---|---|---|---|---|
| $C_{29}H_{39}N_5O_2$ | | (489,66) | | | | |
| Ber.: | C | 71,14 | H | 8,03 | N | 14,30 |
| Gef.: | | 71,04 | | 8,03 | | 14,46 |

Beispiel 12

ll-[4-[3-[4-(Diethylamino)butyl]-l-piperidinyl]-l-oxobutyl]-5,ll-dihydro-6H-pyrido[2,3-b][l,4]benzodiazepin-6-on

Hergestellt analog Beispiel l aus ll-(4-Chlor-l-oxobutyl)-5,ll-dihydro-6H-pyrido[2,3-b][l,4]benzodiazepin-6-on und 3-[4-(Diethylamino)butyl]piperidin in einer Ausbeute von 4,7 % der Theorie. Farblose Kristalle vom Fp. l06-l08°C (Acetonitril).

| | | | | | | |
|---|---|---|---|---|---|---|
| $C_{29}H_{41}N_5O_2$ | (491,68) | | | | | |
| Ber.: | C | 70,84 | H | 8,40 | N | 14,24 |
| Gef.: | | 70,60 | | 8,14 | | 14,19 |

Beispiel 13

5,ll-Dihydro-ll-[4-[3-[2-(l-pyrrolidinyl)ethyl]-l-piperidinyl]-l-oxobutyl]- 6H-pyrido[2,3-b][l,4]benzodiazepin-6-on

Hergestellt analog Beispiel l aus ll-(4-Chlor-l-oxobutyl)-5,ll-dihydro-6H-pyrido[2,3-b][l,4]benzodiazepin-6-on und 3-[2-(l-Pyrrolidinyl)ethyl]piperidin - jedoch unter Verwendung von Acetonitril anstelle von Dimethylformamid als Lösungsmittel - in einer Ausbeute von 8,5 % der Theorie. Farblose Kristalle vom Fp. l26-l28°C (Acetonitril); $R_F$ 0,47 (Macherey-Nagel, Polygram(R) SIL G/$UV_{254}$, pre-coated plastic sheets for TLC; Fließmittel: Dichlormethan/Methanol/Cyclohexan/konz. Ammoniak 68/l5/l5/2 v/v/v/v).

| | | | | | | |
|---|---|---|---|---|---|---|
| $C_{27}H_{35}N_5O_2$ | (461,61) | | | | | |
| Ber.: | C | 70,25 | H | 7,64 | N | 15,17 |
| Gef.: | | 69,95 | | 7,80 | | 15,08 |

Beispiel 14

5,ll-Dihydro-ll-[4-[3-[4-(l-pyrrolidinyl)butyl]-l-piperidinyl]-l-oxobutyl]- 6H-pyrido[2,3-b][l,4]benzodiazepin-6-on

Hergestellt analog Beispiel l aus ll-(4-Chlor-l-oxobutyl]-5,ll-dihydro-6H-pyrido[2,3-b][l,4]benzodiazepin-6-on und 3-[4-(l-Pyrrolidinyl)butyl]-piperidin in einer Ausbeute von l5,5 % der Theorie. Farblose Kristalle vom Fp. ll0-ll2°C (Acetonitril).

| | | | | | | |
|---|---|---|---|---|---|---|
| $C_{29}H_{39}N_5O_2$ | (489,66) | | | | | |
| Ber.: | C | 71,14 | H | 8,03 | N | 14,30 |
| Gef.: | | 70,97 | | 8,08 | | 14,40 |

Beispiel 15

ll-[4-[3-[(Diethylamino)methyl]-4-morpholinyl]-l-oxobutyl]-5,ll-dihydro-6H-pyrido[2,3-b][l,4]benzodiazepin-6-on

Man erwärmte ein Gemisch von l6,33 g (0,0632 Mol) 3-[(Diethylamino)methyl]-4-morpholinbutansäure und 2,0 g einer 75 %igen Natriumhydrid-Dispersion in Paraffinöl in l60 ml wasserfreiem Dimethylformamid bei 50 bis 80°C so lange, bis die Wasserstoffentwicklung beendet war. Zu dem entstandenen Natriumsalz der genannten Säure fügte man l3,20 g (0,0625 Mol) 5,ll-Dihydro-6H-pyrido[2,3-b][l,4]benzodiazepin-6-on und tropfte bei -l0°C 9,9 g (0,0646 Mol) Phosphoroxidchlorid innerhalb von l0 Minuten zu. Man rührte 4 Stunden bei -l0°C, 4 Stunden bei 0°C und 20 Stunden bei Zimmertemperatur. Man rührte die Mischung in 300 g Eis ein, stellte mit Natronlauge auf pH 9 und schüttelte erschöpfend mit Dichlormethan aus. Die vereinigten organischen Phasen wurden einmal mit wenig Eiswasser gewaschen, über Natrium sulfat getrocknet und eingedampft. Der ölige Rückstand wurde mit wenigen Tropfen Diisopropylether verrieben, wobei Kristallisation eintrat. Der Niederschlag wurde abgenutscht und aus t-Butyl-methylether umkristallisiert. Man erhielt 6,55 g (23 % der Theorie) an farblosen Kristallen om Fp. l08-ll0°C, nach Dünnschichtchromatogramm, Mischschmelzpunkt, IR, UV- und $^lH$-NMR-Spektrum völlig identisch mit einer nach Beispiel 5 erhaltenen Probe.

Im folgenden wird die Herstellung pharmazeutischer Anwendungsformen anhand einiger Beispiele beschrieben:

Beispiel I

Tabletten mit 5 mgll-[4-[3-[(Diethylamino)]methyl]-4-morpholinyl]-l-oxobutyl]-5,ll-dihydro-6H-pyrido[2,3- b][l,4]-benzodiazepin-6-on

Zusammensetzung:
1 Tablette enthält:

| | |
|---|---|
| Wirkstoff | 5,0 mg |
| Milchzucker | 148,0 mg |
| Kartoffelstärke | 65,0 mg |
| Magnesiumstearat | 2,0 mg |
| | 220,0 mg |

Herstellungsverfahren:

Aus Kartoffelstärke wird durch Erwärmen ein l0%iger Schleim hergestellt. Die Wirksubstanz, Milchzucker und die restliche Kartoffelstärke werden gemischt und mit obigem Schleim durch ein Sieb der Maschenweite l,5 mm granuliert. Das Granulat wird bei 45°C getrocknet, nochmals durch obiges Sieb gerieben, mit Magnesiumstearat vermischt und zu Tabletten verpreßt.

| | |
|---|---|
| Tablettengewicht: | 220 mg |
| Stempel: | 9 mm |

Beispiel II

Dragées mit 5 mg ll-[4-[3-[(Diethylamino)methyl]-4-morpholinyl]-l-oxobutyl]-5,ll-dihydro-6H-pyrido[2,3-b][l,4]benzodiazepin-6-on

Die nach Beispiel I hergestellten Tabletten werden nach bekanntem Verfahren mit einer Hülle überzogen, die im wesentlichen aus Zucker und Talkum besteht. Die fertigen Dragées werden mit Hilfe von Bienenwachs poliert.
Dragéegewicht: 300 mg

Beispiel III

Ampullen mit l0 mg 11-[4-[3-[(Diethylamino)methyl]-4-morpholinyl]-1-oxobutyl]-5,11-dihydro-6H-pyrido[2,3-b][1,4]benzodiazepin-6-on

Zusammensetzung:
1 Ampulle enthält:

| | | |
|---|---|---|
| Wirkstoff | | 10,0 mg |
| Natriumchlorid | | 8,0 mg |
| Dest. Wasser | ad | 1 ml |

Herstellungsverfahren:

Die Wirksubstanz und Natriumchlorid werden in dest. Wasser gelöst und anschließend auf das gegebene Volumen aufgefüllt. Die Lösung wird sterilfiltriert und in l ml-Ampullen abgefüllt.
Sterilisation: 20 Minuten bei l20°C.

Beispiel IV

Suppositorien mit 20 mg ll-[4-[3-[(Diethylamino)methyl]-4-morpholinyl]-l-oxobutyl]-5,ll-dihydro-6H-pyrido[2,3-b][l,4]benzodiazepin-6-on

Zusammensetzung:
1 Zäpfchen enthält:

| | |
|---|---|
| Wirkstoff | 20,0 mg |
| Zäpfchenmasse (z.B. Witepsol W 45(R)) | 1 680,0 mg |
| | 1 700,0 mg |

Herstellungsverfahren:

Die feinpulverisierte Wirksubstanz wird in der geschmolzenen und auf 40°C abgekühlten Zäpfchenmasse suspendiert. Man gießt die Masse bei 37°C in leicht vorgekühlte Zäpfchenformen aus.
Zäpfchengewicht l,7 g

Beispiel V

Tropfen mit ll-[4-[3-[(Diethylamino)methyl]-4-morpholinyl]-l-oxobutyl]-5,ll-dihydro-6H-pyrido[2,3- b][l,4]benzodiazepin-6-on

Zusammensetzung:
100 ml Tropflösung enthalten:

| | | | |
|---|---|---|---|
| p-Hydroxybenzoesäuremethylester | | 0,035 | g |
| p-Hydroxybenzoesäurepropylester | | 0,015 | g |
| Anisöl | | 0,05 | g |
| Menthol | | 0,06 | g |
| Ethanol rein | | 10,0 | g |
| Wirkstoff | | 0,5 | g |
| Natriumcyclamat | | 1,0 | g |
| Glycerin | | 15,0 | g |
| Dest. Wasser | ad | 100,0 | ml |

Herstellungsverfahren:

Die Wirksubstanz und Natriumcyclamat werden in ca. 70 ml Wasser gelöst und Glycerin zugefügt. Man löst p-Hydroxybenzoesäureester, Anisöl sowie Menthol in Ethanol und fügt diese Lösung unter Rühren der wäßrigen Lösung zu. Abschließend wird mit Wasser auf l00 ml aufgefüllt und schwebeteilchenfrei filtriert.

## Patentansprüche

### Patentansprüche für folgende Vertragsstaaten : At, BE, CH, DE, FR, GB, IT, LU, NL, SE

I.) Kondensierte Diazepinone der allgemeinen Formel I,

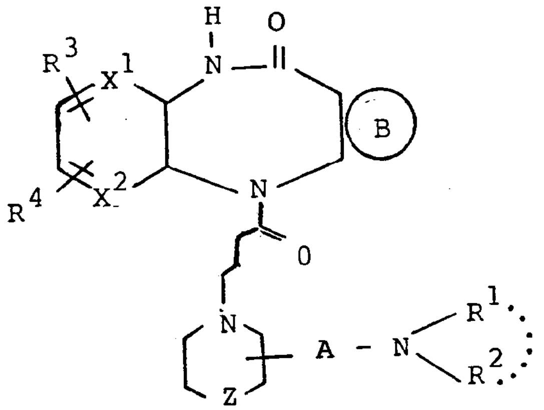

(I)

in der

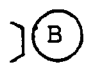 einen der zweiwertigen Reste

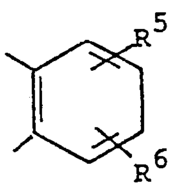 , 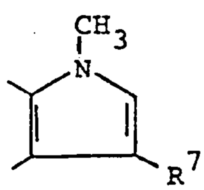 , 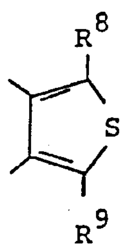 , 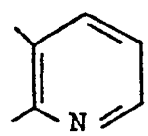 oder 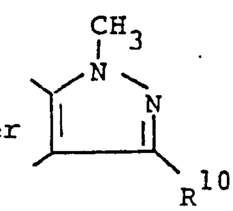

(S) (T) (U) (V) (W)

darstellt und
$X^1$, $X^2$, A, $R^1$ bis $R^{10}$ und Z die folgenden Bedeutungen besitzen:

$X^1$ und $X^2$ stellen eine =CH-Gruppe dar oder, sofern 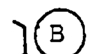 die Bedeutungen der oben genannten zweiwertigen Reste (S), (U) oder (W) annimmt, können beide oder nur $X^1$ oder nur $X^2$ auch ein Stickstoffatom darstellen,

A ist ein geradkettiger, gesättigter Alkylenrest mit I bis 6 Kohlenstoffatomen;

$R^1$ und $R^2$ sind gleiche oder voneinander verschiedene Alkylreste mit bis zu 6 Kohlenstoffatomen oder bedeuten zusammen mit dem dazwischenliegenden Stickstoffatom einen 4- bis 7-gliedrigen, gesättigten, monocyclischen, heteroaliphatischen Ring, der gegebenenfalls durch ein Sauerstoffatom oder durch die $N\text{-}CH_3$-Gruppe unterbrochen sein kann;

$R^3$ ist eine Alkylgruppe mit I bis 4 C-Atomen, ein Chloratom oder ein Wasserstoffatom;

$R^4$ ein Wasserstoffatom oder eine Methylgruppe;

$R^5$ und $R^6$ sind jeweils ein Wasserstoffatom ein Fluor-, Chlor- oder Bromatom oder eine Alkylgruppe mit I bis 4 C-Atomen;

$R^7$ ist ein Wasserstoff- oder Chloratom oder eine Methylgruppe;

$R^8$ ein Wasserstoffatom oder ein Alkylrest mit I bis 4 Kohlenstoffatomen;

$R^9$ ein Wasserstoff- oder Halogenatom oder eine Alkylgruppe mit I bis 4 Kohlenstoffatomen;

$R^{10}$ ein Wasserstoffatom oder eine Methylgruppe und
Z eine Einfachbindung, ein Sauerstoff- oder Schwefelatom, die Methylen- oder I,2-Ethylengruppe;

wobei, sofern 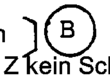 den zweiwertigen Rest (T) darstellt und $R^7$ ein Wasserstoffatom bedeutet, $R^3$ kein Chloratom und Z kein Schwefelatom bedeuten können;

gegebenenfalls ihre diastereomeren bzw. enantiomeren Formen, und ihre physiologisch verträglichen Salze mit anorganischen oder organischen Säuren.

2.) Kondensierte Diazepinone der allgemeinen Formel I gemäß Anspruch I, worin

$X^1$ eine =CH-Gruppe und

$X^2$ entweder ein Stickstoffatom und 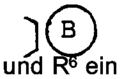 den zweiwertigen Rest (S) darstellt, mit der Maßgabe, daß $R^3$, $R^4$ und $R^5$ Wasserstoffatome sind und $R^6$ ein Wasserstoffatom, ein Chlor- oder Bromatom oder eine Methyl- oder Ethylgruppe in 8- oder 9-Stellung des Heterocyclus ist,

oder eine =CH-Gruppe darstellt, wenn 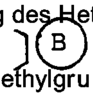 die Bedeutung der zweiwertigen Reste V oder U annimmt, $R^8$ ein Wasserstoffatom und $R^9$ eine Methylgruppe ist;

A eine unverzweigte, gesättigte Alkylenkette mit I bis 5 Kohlenstoffatomen bedeutet;

$R^1$ und $R^2$ gleiche oder voneinander verschiedene Alkylreste mit I bis 5 Kohlenstoffatomen oder zusammen mit dem eingeschlossenen Stickstoffatom einen 4- bis 7-gliedrigen, gesättigten, monocyclischen, heteroaliphatischen Ring darstellen, der gegebenenfalls durch ein Sauerstoffatom oder durch die $N-CH_3$-Gruppe unterbrochen sein kann

und

Z eine Einfachbindung, ein Sauerstoffatom, die Methylenoder I,2-Ethylengruppe ist und, gegebenenfalls, ihre diastereomeren bzw. enantiomeren Formen, und ihre physiologisch verträglichen Salze mit anorganischen oder organischen Säuren.

3.) Kondensierte Diazepinone der allgemeinen Formel I gemäß Anspruch I und 2, worin

$X^1$ eine =CH-Gruppe;

$X^2$ ein Stickstoffatom und 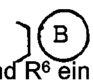 den zweiwertigen Rest (S) bedeutet, mit der Maßgabe, daß $R^3$, $R^4$ und $R^5$ Wasserstoffatome sind und $R^6$ ein Wasserstoffatom ein Chlor- oder Bromatom oder eine Methyl- oder Ethylgruppe in 8- oder 9-Stellung des Heterocyclus ist,

A eine unverzweigte, gesättigte Alkylenkette mit I bis 5 Kohlenstoffatomen bedeutet,

$R^1$ und $R^2$ gleiche oder voneinander verschiedene Alkylreste mit I bis 5 Kohlenstoffatomen oder zusammen mit dem dazwischenliegenden Stickstoffatom einen 5- bis 7-gliedrigen, gesättigten, monocyclischen, heteroaliphatischen Ring darstellen, der gegebenenfalls durch ein Sauerstoffatom oder durch die $>N-CH_3$-Gruppe unterbrochen sein kann

und

Z eine Einfachbindung, ein Sauerstoffatom, die Methylenoder I,2-Ethylengruppe ist,
und gegebenenfalls, ihre diastereomeren bzw. enantiomeren Formen, und ihre physiologisch verträglichen Salze mit anorganischen oder organischen Säuren.

4.) Als kondensierte Diazepinone gemäß den Ansprüchen I bis 3 die Verbindungen

II-[4-[3-[(Diethylamino)methyl]-4-morpholinyl]-I-oxobutyl]-5,II-dihydro-6H-pyrido[2,3-b][I,4]benzodiazepin-6-on;

II-[4-[2-[(Diethylamino)methyl]-I-piperidinyl]-I-oxobutyl]-5,II-dihydro-9-methyl-6H-pyrido[2,3-b][I,4]benzodiazepin-6-on

5,II-Dihydro-II-[4-[2-[(I-pyrrolidinyl)methyl]-I-piperidinyl]-I-oxobutyl]-6H-pyrido[2,3-b][I,4]benzodiazepin-6-on

und ihre physiologisch verträglichen Salze mit anorganischen oder organischen Säuren.

5.) Arzneimittel enthaltend eine oder mehrere Verbindungen der Ansprüche I bis 4 neben den üblichen Träger- und/oder Hilfsstoffen.

6.) Verwendung der Verbindungen der Ansprüche I bis 4 zur Herstellung von Arzneimitteln zur Behandlung von Bradycardien und Bradyarrhythmien und, gegebenenfalls, von Spasmen an der Blase und dem Colon.

7.) Verfahren zur Herstellung von kondensierten Diazepinonen der allgemeinen Formel I, gemäb Anspruch 1 gegebenenfalls von ihren diastereomeren bzw. enantiomeren Formen, und von ihren Salzen mit anorganischen oder organischen Säuren, dadurch gekennzeichnet, daß

a.) zur Herstellung von basisch substituierten kondensierten Diazepinonen der allgemeinen Formel Ia,

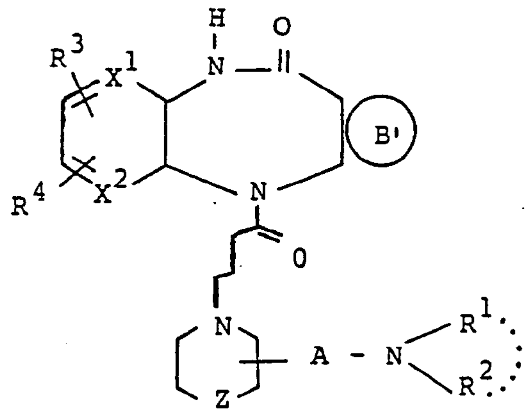

(Ia)

in der

$R^I$, $R^2$, $R^3$, $R^4$, $X^1$, $X^2$, A und Z die oben angegebenen Bedeutungen haben und 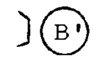 einen der zweiwertigen Reste (S), (U), (V), (W) oder (T′)

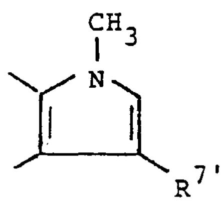

(T')

darstellt, wobei $R^{7'}$ ein Chloratom oder eine Methylgruppe ist,

eine Halogenacylverbindungen der allgemeinen Formel II,

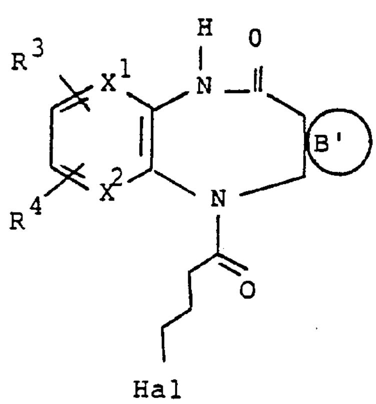

(II)

in der

$R^3$, $R^4$, 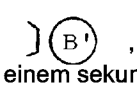, $X^I$ und $X^2$ die angegebenen Bedeutungen haben und Hal ein Chlor-, Brom- oder Iodatom ist, mit einem sekundären Amin der allgemeinen Formel III,

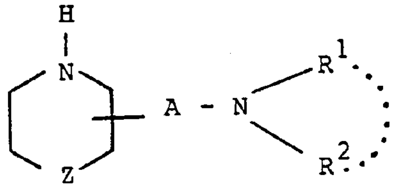

(III)

in der
$R^l$, $R^2$, A und Z wie oben genannt definiert sind, in einem inerten Lösungsmittel, bevorzugt in Gegenwart einer Hilfsbase oder eines Überschusses des Amins der allgemeinen Formel III, bei Temperaturem zwischen -l0°C und der Siedetemperaturdes Reaktionsgemisches umgesetzt wird oder

b.) zur Herstellung von basisch substituierten kondensierten Diazepinonen der allgemeinen Formel Ia mit den vorstehend genannten Bedeutungen der Substituenten Tricyclen der allgemeinen Formel IV,

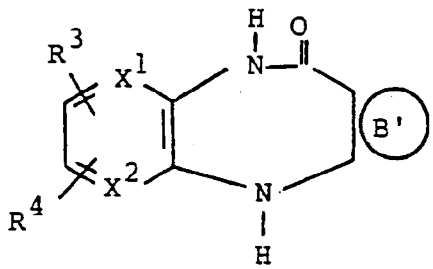

(IV)

in der
die Reste $R^3$, $R^4$, $X^l$, $X^2$ und 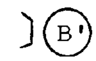 wie oben definiert sind, mit Carbonsäurederivaten der allgemeinen Formel V,

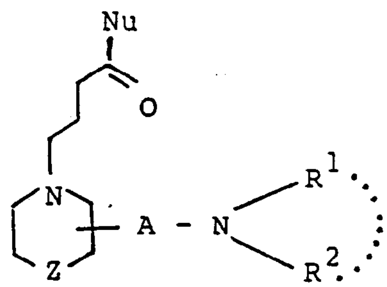

(V)

worin
$R^l$, $R^2$, A und Z die oben angegebenen Bedeutungen haben und Nu eine nucleofuge Gruppe bzw. Abgangsgruppe darstellt, in einem inerten Lösungsmittel bei Temperatur en zwischen -25 und l30°C, gegebenenfalls in Gegenwart eines Protonenakzeptors, umgesetzt werden oder

c.) zur Herstellung eines unter die allgemeine Formel I fallenden Pyrrolo-kondensierten Diazepinons der allgemeinen Formel Ib,

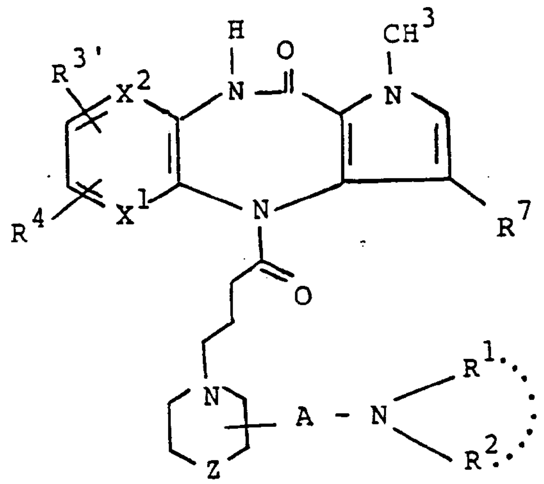

(Ib)

worin

$R^1$, $R^2$, $R^4$, $X^1$, $X^2$ und A wie oben angegeben definiert sind, Z die eingangs erwähnten Bedeutungen mit Ausnahme der eines Schwefelatoms hat, $R^{3'}$ eine Alkylgruppe mit 1 bis 4 Kohlenstoffatomen oder ein Wasserstoffatom ist und $R^7$ ein Wasserstoffatom darstellt, eine solche Verbindung der allgemeinen Formel Ib, in der $R^7$ ein Chloratom bedeutet, in einem Lösungsmittel entweder in Gegenwart von Katalysatoren von Metallen der VIII. Nebengruppe des Perioden systems der Elemente bei Wasserstoffdrucken von 1 bis 300 bar und Temperaturen von 0°C bis 130°C oder mit Ameisensäure in Gegenwart von Palladium auf Kohle als Katalysator be Temperaturen zwischen 70 und 110°C oder mit Triethylammoniumformiat in Gegenwart überschüssigen Triethylamins und von Palladium auf Tierkohle oder von Palladiumacetat und Triarylphosphinen, bei Temperaturen zwischen 40 und 110°C hydrogenolysiert wird und gegebenenfalls, so erhaltene Verbindungen der allgemeinen Formel I in ihre diastereomeren bzw. enantiomeren Formen aufgetrennt und/oder so erhaltene Verbindungen der allgemeinen Formel I in ihre Salze, vorzugsweise in ihre physiologisch verträglichen Salze mit anorganischen oder organischen Säuren, übergeführt werden.

8. Verfahren gemäß Anspruch 7b, dadurch gekennzeichnet, daß als Carbonsäurederivate der allgemeinen Formel V Säurehalogenide, -ester, -anhydride oder gemischte Säureanhydride eingesetzt werden.

**Patentansprüche für folgende Vertragsstaaten: ES, GR**

1.) Verfahren zur Herstellung von kondensierten Diazepinonen der allgemeinen Formel I,

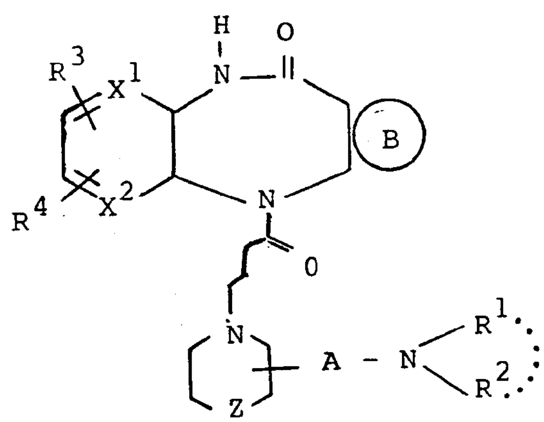

(I)

in der

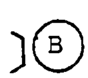 einen der zweiwertigen Reste

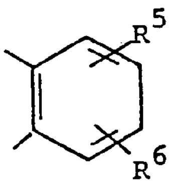, 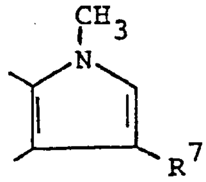, 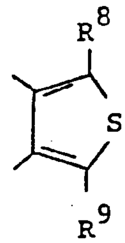, 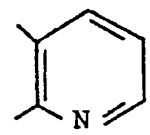 oder 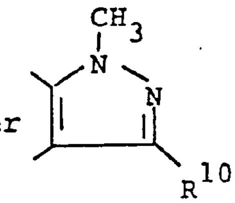

(S) (T) (U) (V) (W)

darstellt und
$X^1$, $X^2$, A, $R^1$ bis $R^{10}$ und Z die folgenden Bedeutungen besitzen:

$X^1$ und $X^2$ stellen eine =CH-Gruppe dar oder sofern 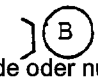 die Bedeutungen der oben genannten zweiwertigen Reste (S), (U) oder (W) annimmt, können beide oder nur $X^1$ oder nur $X^2$ auch ein Stickstoffatom darstellen,

A ist ein geradkettiger, gesättigter Alkylenrest mit l bis 6 Kohlenstoffatomen;

$R^1$ und $R^2$ sind gleiche oder voneinander verschiedene Alkylreste mit bis zu 6 Kohlenstoffatomen oder bedeuten zusammen mit dem dazwischenliegenden Stickstoffatom einen 4- bis 7-gliedrigen, gesättigten, monocyclischen, heteroaliphatischen Ring, der gegebenenfalls durch ein Sauerstoffatom oder durch die N-$CH_3$-Gruppe unterbrochen sein kann;

$R^3$ ist eine Alkylgruppe mit l bis 4 C-Atomen, ein Chloratom oder ein Wasserstoffatom;

$R^4$ ein Wasserstoffatom oder eine Methylgruppe;

$R^5$ und $R^6$ sind jeweils ein Wasserstoffatom, ein Fluor-, Chlor- oder Bromatom oder eine Alkylgruppe mit l bis 4 C-Atomen;

$R^7$ ist ein Wasserstoff- oder Chloratom oder eine Methylgruppe;
$R^8$ ein Wasserstoffatom oder ein Alkylrest mit l bis 4 Kohlenstoffatomen;
$R^9$ ein Wasserstoff- oder Halogenatom oder eine Alkylgruppe mit l bis 4 Kohlenstoffatomen und
$R^{10}$ ein Wasserstoffatom oder eine Methylgruppe und
Z eine Einfachbindung, ein Sauerstoff- oder Schwefelatom, die Methylen- oder l,2-Ethylengruppe;

wobei, sofern 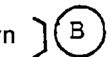 den zweiwertigen Rest (T) darstellt und $R^7$ ein Wasserstoffatom bedeutet, $R^3$ kein Chloratom und Z kein Schwefelatom bedeuten können;

gegebenenfalls von ihren diastereomeren bzw. enantiomeren Formen, und von ihren Salzen mit anorganischen oder organischen Säuren, dadurch gekennzeichnet, daß

a.) zur Herstellung von basisch substituierten kondensierten Diazepinonen der allgemeinen Formel Ia,

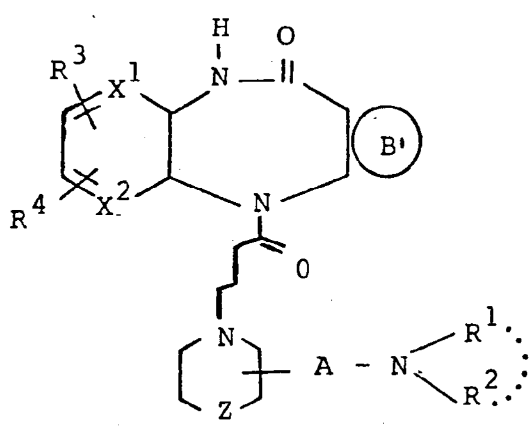

(Ia)

in der

$R^1$, $R^2$, $R^3$, $R^4$, $X^1$, $X^2$, A und Z die oben angegebenen Bedeutungen haben und 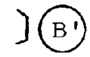 einen der zweiwertigen Reste (S), (U), (V), (W) oder (T')

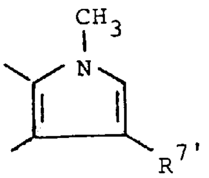

(T')

darstellt, wobei $R^{7'}$ ein Chloratom oder eine Methylgruppe ist,

eine Halogenacylverbindungen der allgemeinen Formel II,

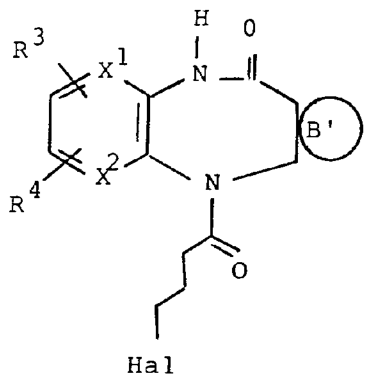

(II)

in der

$R^3$, $R^4$, 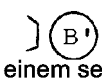 , $X^1$ und $X^2$ die angegebenen Bedeutungen haben und Hal ein Chlor-, Brom- oder Iodatom ist, mit einem sekundären Amin der allgemeinen Formel III,

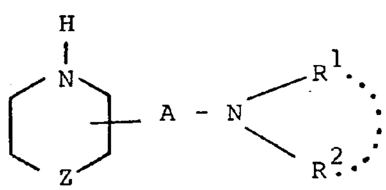

(III)

in der

$R^1$, $R^2$, A und Z wie oben genannt definiert sind, in einem inerten Lösungsmittel, bevorzugt in Gegenwart einer Hilfsbase oder eines Überschusses des Amins der allgemeinen Formel III, bei Temperaturem zwischen -10° und der Siedetemperatur des Reaktionsgemisches umgesetzt wird oder

b.) zur Herstellung von basisch substituierten kondensierten Diazepinonen der allgemeinen Formel Ia mit den vorstehend genannten Bedeutungen der Substituenten Tricyclen der allgemeinen Formel IV,

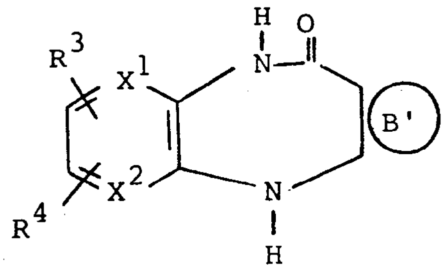

(IV)

in der

die Reste $R^3$, $R^4$, $X^1$, $X^2$ und 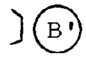 wie oben definiert sind, mit Carbonsäurederivaten der allgemeinen Formel V,

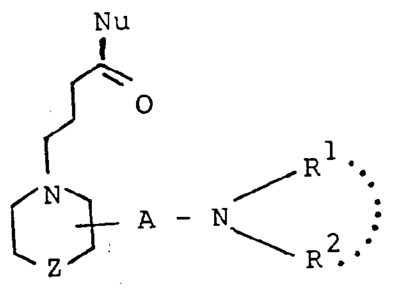

(V)

worin
$R^1$, $R^2$, A und Z die oben angegebenen Bedeutungen haben und Nu eine nucleofuge Gruppe bzw. Abgangsgruppe darstellt, in einem inerten Lösungsmittel bei Temperaturen zwischen -25 und 130°C, gegebenenfalls in Gegenwart eines Protonenakzeptors, umgesetzt werden oder

c.) zur Herstellung eines unter die allgemeine Formel I fallenden Pyrrolo-kondensierten Diazepinons der allgemeinen Formel Ib,

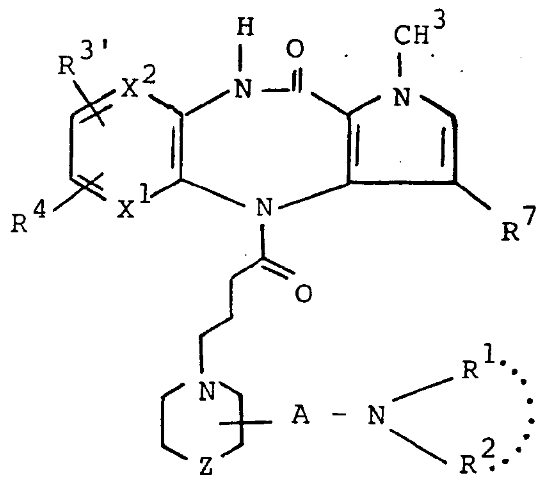

(Ib)

worin
$R^1$, $R^2$, $R^4$, $X^1$, $X^2$ und A wie oben angegeben definiert sind, Z die eingangs erwähnten Bedeutungen mit Ausnahme der eines Schwefelatoms hat, $R^{3'}$ eine Alkylgruppe mit 1 bis 4 Kohlenstoffatomen oder ein Wasserstoffatom ist und $R^7$ ein Wasserstoffatom darstellt, eine solche Verbindung der allgemeinen Formel Ib, in der $R^7$ ein Chloratom bedeutet, in einem Lösungsmittel entweder in Gegenwart von Katalysatoren von Metallen der VIII. Nebengruppe des Periodensystems der Elemente bei Wasserstoffdrucken von 1 bis 300 bar und Temperaturen von 0°C bis 130°C oder mit Ameisensäure in Gegenwart von Palladium auf Kohle als Katalysator bei Temperaturen zwischen 70 und 110°C oder mit Triethylammoniumformiat in Gegenwart überschüssigen Triethylamins und von Palladium auf Tierkohle oder von Palladiumacetat und Triarylphosphinen, bei Temperaturen zwischen 40 und II0°C hydrogenolysiert wird

und gegebenenfalls, so erhaltene Verbindungen der allgemeinen Formel I in ihre diastereomeren bzw. enantiomeren Formen aufgetrennt und/oder so erhaltene Verbindungen der allgemeinen Formel I in ihre Salze, vorzugsweise in ihre physiologisch verträglichen Salze mit anorganischen oder organischen Säuren, übergeführt werden.

2. Verfahren gemäß Anspruch Ib, dadurch gekennzeichnet, daß als Carbonsäurederivate der allgemeinen Formel V Säurehalogenide, -ester, -anhydride oder gemischte Säureanhydride eingesetzt werden.

## Claims

### Claims for the following Contracting States : AT, BE, CH, DE, FR, GB, IT, LU, NL, SE

1. Condensed diazepinones of general formula I

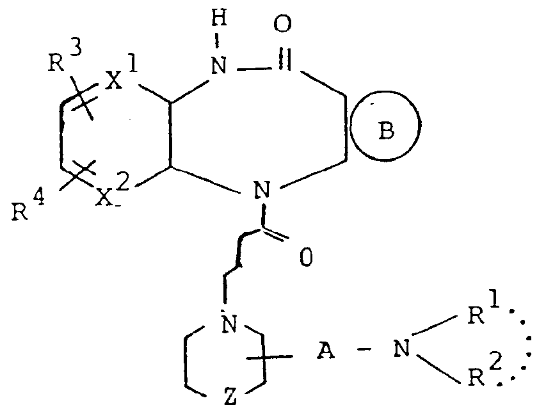

(I)

wherein
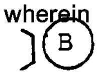 represents one of the divalent groups

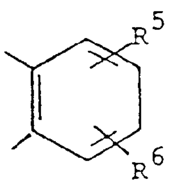 , 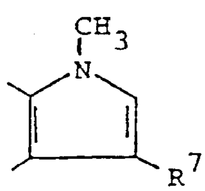 , 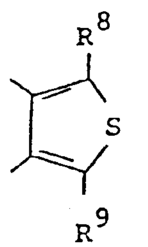 , 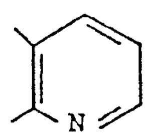 or 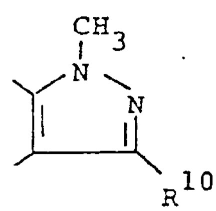

(S) (T) (U) (V) (W)

and
$X^1$, $X_2$, A, $R^1$ to $R^{10}$ and Z are defined as follows:

$X^1$ and $X^2$ represent a =CH group or, if 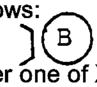 assumes the meanings of the above-mentioned divalents groups (S), (U) or (W), both or either one of $X^1$ and $X^2$ may also represent a nitrogen atom;

A is a straight-chained saturated alkylene group having 1 to 6 carbon atoms;

$R^1$ and $R^2$ are identical or different alkyl groups having up to 6 carbon atoms or, together with the intervening nitrogen atom, represent a 4- to 7-membered, saturated, monocyclic, heteroaliphatic ring which may optionally be interrupted by an oxygen atom or by an $N-CH_3$ group;

$R^3$ is an alkyl group having 1 to 4 carbon atoms, a chlorine atom or a hydrogen atom;

$R^4$ is a hydrogen atom or a methyl group;

$R^5$ and $R^6$ each represents a hydrogen atom, a fluorine, chlorine or bromine atom or an alkyl group having 1 to 4 carbon atoms;

$R^7$ is a hydrogen or chlorine atom or a methyl group;

$R^8$ is a hydrogen atom or an alkyl group having 1 to 4 carbon atoms;
$R^9$ is a hydrogen or halogen atom or an alkyl group having 1 to 4 carbon atoms and
$R^{10}$ is a hydrogen atom or a methyl group and
Z is a single bond, an oxygen or sulphur atom or a methylene or 1,2-ethylene group;
whilst, if 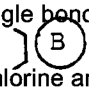 represents the divalent group (T) and $R^7$ represents a hydrogen atom, $R^3$ cannot represent chlorine and Z cannot represent sulphur;

and optionally the diastereomeric or enantiomeric forms thereof, and the physiologically acceptable salts thereof with inorganic or organic acids.

2. Condensed diazepinones of general formula I according to claim 1, wherein
$X^1$ represents a =CH group,
$X^2$ either represents a nitrogen atom and 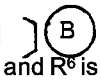 represents the divalent group (S), with the proviso that $R^3$ , $R^4$and $R^5$are hydrogen atoms and $R^6$ is a hydrogen atom, a chlorine or bromine atom or a methyl or ethyl group in the 8- or 9- position of the heterocycle or it represents a =CH group, if 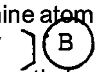 assumes the meaning of the divalent groups V or U, whilst $R^8$ is a hydrogen atom and $R^9$ is a methyl group;
A represents an unbranched, saturated alkylene chain having 1 to 5 carbon atoms;
$R^1$ and $R^2$ represent identical or different alkyl groups having 1 to 5 carbon atoms or, together with the intervening nitrogen atom, they represent a 4- to 7- membered, saturated, monocyclic, heteroaliphatic ring which may optionally be interrupted by an oxygen atom or by the N-CH3 group
and
Z is a single bond, an oxygen atom or a methylene or 1,2-ethylene group and optionally the diastereomeric or enantiomeric forms thereof, and the physiologically acceptable salts thereof with inorganic or organic acid.

3. Condensed diazepinones of general formula I according to claims 1 and 2, wherein
$X^1$ represents a =CH group;
$X^2$ represents a nitrogen atom and 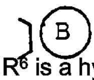 reprensents the divalent group (S), with the proviso that $R^3$, $R^4$ and $R^5$ are hydrogen atoms and $R^6$ is a hydrogen atom, a chlorine or bromine atom or a methyl or ethyl group in the 8- or 9-position of the heterocycle,
A represents an unbranched, saturated alkylene chain having 1 to 5 carbon atoms,
$R^1$ and $R^2$ represent identical or different alkyl groups having 1 to 5 carbon atoms or, together with the intervening nitrogen atom, represent a 5- to 7-membered, saturated, monocyclic, heteroaliphatic ring which may optionally be interrupted by an oxygen atom or by the $>N-CH^3$ group
and
Z represents a single bond, an oxygen atom or a methylene or 1,2-ethylene group and optionally the diastereomeric or enantiomeric forms thereof, and the physiologically acceptable salts thereof with inorganic or organic acids.

4. As condensed diazepinones according to claims 1 to 3, the compounds
11-[4-[3-[(diethylamino)methyl]-4-morpholinyl]-1oxobutyl]-5,11-dihydro-6H-pyrido[2,3-b][1,4]benzodiazepin-6-one;
11-[4-[2-[(diethylamino)methyl]-1-piperidinyl]-1oxobutyl]-5,11-dihydro-9-methyl-6H-pyrido[2,3-b][1,4]benzodiazepin-6-one;
5,11-dihydro-11-[4-[2-[(1-pyrrolidinyl)methyl]-1piperidinyl]-1-oxobutyl]-6H-pyrido[2,3-b][1,4]benzodiazepin-6-one
and the physiologically acceptable salts thereof with inorganic or organic acids.

5. Pharmaceutical compositions containing one or more compounds according to claims 1 to 4 in addition to the conventional carriers and/or excipients.

6. Use of the compounds of claims 1 to 4 for preparing pharmaceutical compositions for the treatment of bradycardia and bradyarrhythmia and optionally spasm in the bladder and colon.

7. Process for preparing condensed diazepinones of general formula I according to claim 1 and optionally the diastereoineric or enantiomeric forins thereof, and the physiologically acceptable salts thereof with inorganic or organic acids, characterised in that
a) in order to prepare base-substituted condensed diazepinones of general formula Ia

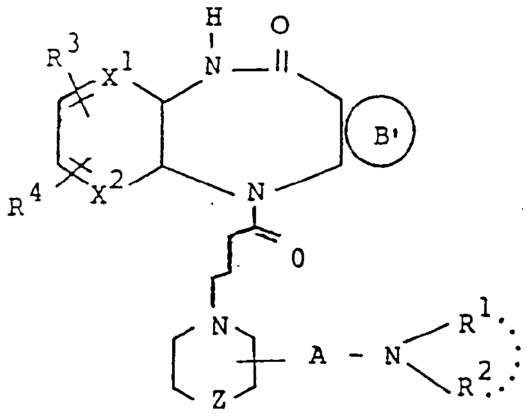

(Ia)

wherein
$R^1$, $R^2$, $R^3$, $R^4$, $X^1$ $X^2$, A and Z are defined as hereinbefore and 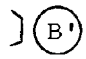 represents one of the divalent groups (S), (U), (V), (W), or (T′)

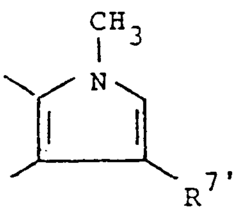

(T')

wherein $R^{7'}$ ,is a chlorine atom or a methyl group,
a haloacyl compound of general formula II

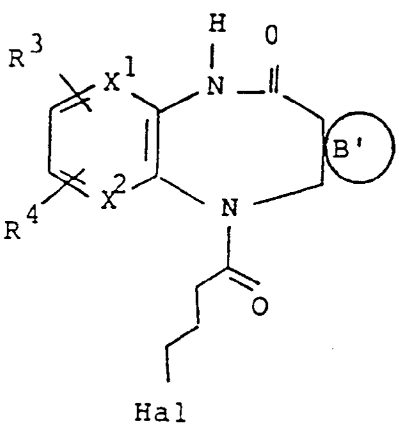

(II)

wherein
$R^3$, $R^4$, 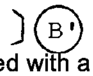 , $X^1$ and $X^2$ are defined as hereinbefore and Hal is a chlorine, bromine or iodine atom, is reacted with a secondary amine of general formula III

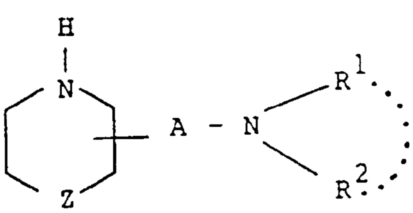

(III)

wherein
$R^1$ , $R^2$, A and Z are defined as hereinbefore, in an inert solvent, preferably in the presence of an auxiliary

base or an excess of the amine of general formula III, at temperatures of between -10°C and the boiling temperature of the reaction mixture or

b) in order to prepare base-substituted condensed diazepinones of general formula Ia in which the substituents are defined as hereinbefore, tricyclic compounds of general formula IV

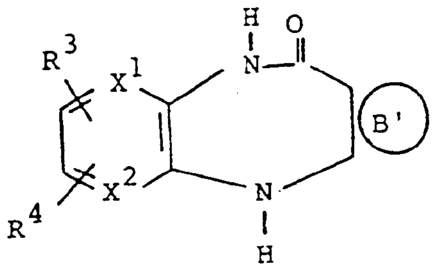

(IV)

wherein
the groups $R^3$, $R^4$, $X^1$, $X^2$ and 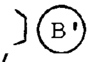 are defined as hereinbefore, are reacted with carboxylic acid derivatives of general formula V

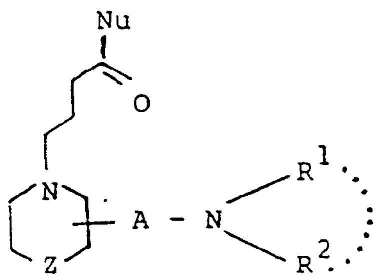

(V)

wherein
$R^1$, $R^2$, A and Z are defined as hereinbefore and Nu represents a nucleofugic group or leaving group, in an inert solvent at temperatures of between -25 and 130°C, optionally in the presence of a proton acceptor, or

c) in order to prepare a pyrrolo-condensed diazepinone of general formula Ib covered by general formula I,

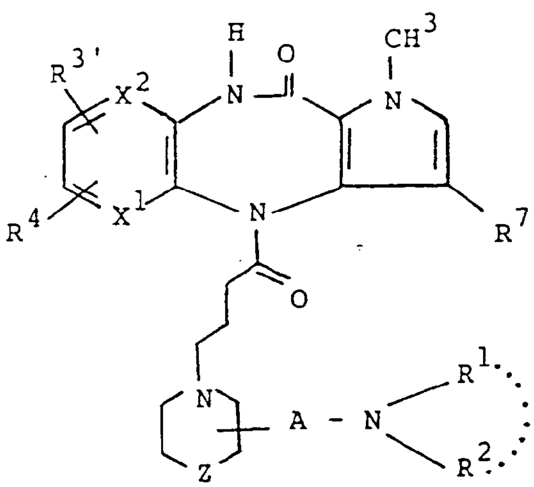

(Ib)

wherein
$R^1$, $R_2$, $R^4$, $X^1$, $X^2$ and A are defined as hereinbefore, Z has the meanings given hereinbefore with the exception of a sulphur atom, $R^{3'}$ is an alkyl group having 1 to 4 carbon atoms or a hydrogen atom and $R^7$ represents a hydrogen atom,
a compound of general formula Ib wherein $R^7$ represents a chlorine atom is hydrogenolysed in a solvent

with hydrogen, either in the presence of catalysts of metals of the VIIIth sub-group of the periodic Table of Elements under hydrogen pressures of from 1 to 300 bar and at temperatures of from 0°C to 130°C or with formic acid in the presence of palladium on charcoal as catalyst at temperatures of between 70 and 110°C or with triethylammonium formate in the presence of excess triethylamine and palladium on animal charcoal or palladium acetate and triarylphosphines, at temperatures of between 40 and 110°C
and if desired compounds of general formula I thus obtained are separated into their diastereoisomeric or enantiomeric forms and/or compounds of general formula I thus obtained are converted into their salts, preferably their physiologically acceptable salts with inorganic or organic acids.

8. Process as claimed in claim 7b, characterised in that acid halides, esters, anhydrides or mixed acid anhydrides are used as the carboxylic acid derivatives of general formula V.

**Claims for the following Contracting States : ES, GR**

1. Process for preparing condensed diazepinones of general formula I

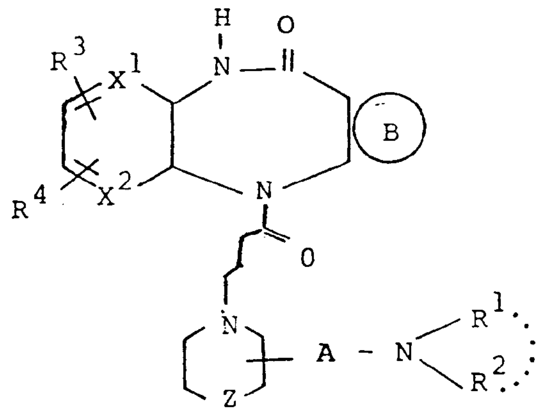

(I)

wherein
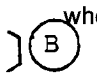 represents one of the divalent groups

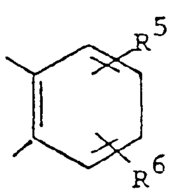 ,
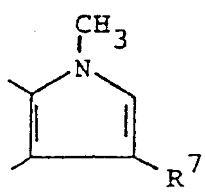 ,
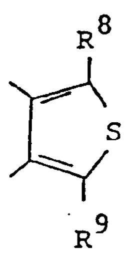 ,
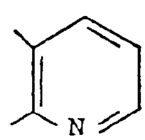 or
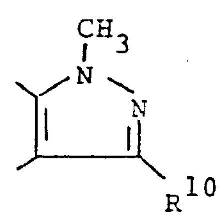

(S) (T) (U) (V) (W)

and
$X^1$ ,$X^2$, A, $R^1$ to $R^{10}$ and Z are defined as follows:

$X^1$ and $X^2$ represent a =CH group or, if 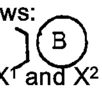 assumes the meaning above-mentioned divalent groups (S), (U) or (W) , both or either one of $X^1$ and $X^2$ may also represent a nitrogen atom;

A is a straight-chained saturated alkylene group having 1 to 6 carbon atoms;

$R^1$ and $R^2$ are identical or different alkyl groups having up to 6 carbon atoms or, together with the intervening nitrogen atom, represent a 4- to 7-membered, saturated, monocyclic, heteroaliphatic ring which may optionally be interrupted by an oxygen atom or by an N-$CH_3$ group;

$R^3$ is an alkyl group having 1 to 4 carbon atoms, a chlorine atom or a hydrogen atom;

$R^4$ is a hydrogen atom or a methyl group;

$R^5$ and $R^6$ each represents a hydrogen atom, a fluorine, chlorine or bromine atom or an alkyl group having 1 to 4 carbon atoms;

$R^7$ is a hydrogen or chlorine atom or a methyl group;

$R^8$ is a hydrogen atom or an alkyl group having 1 to 4 carbon atoms;
$R^9$ is a hydrogen or halogen atom or an alkyl group having 1 to 4 carbon atoms and
$R^{10}$ is a hydrogen atom or a methyl group and
Z is a single bond, an oxygen or sulphur atom or a methylene or 1,2-ethylene group;

whilst, if 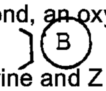 represents the divalent group (T) and $R^7$ represent a hydrogen atom, $R^3$ cannot represent chlorine and Z cannot represent sulphur;

and optionally the diastereomeric or enantiomeric forms thereof, and the physiologically acceptable salts thereof with inorganic or organic acids, characterised in that

a) in order to prepare base-substituted condensed diazepinones of general formula Ia

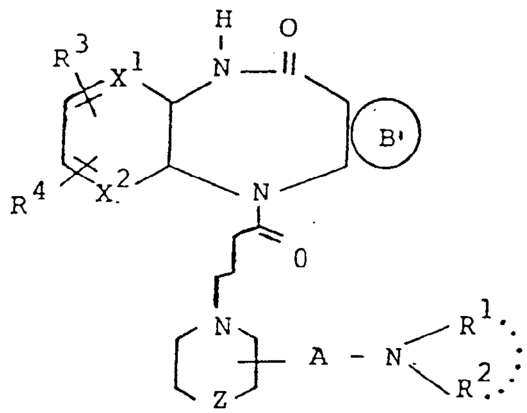

(Ia)

wherein
$R^1$, $R^2$, $R^3$, $R^4$, $X^1$, $X^2$, A and Z are defined as hereinbefore and 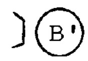 represents one of the divalent groups (S), (U), (V), (W) or (T')

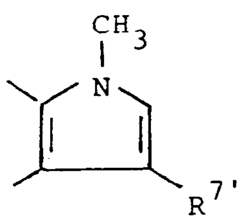

(T')

wherein $R^{7'}$is a chlorine atom or a methyl group,

a haloacyl compound of general formula II

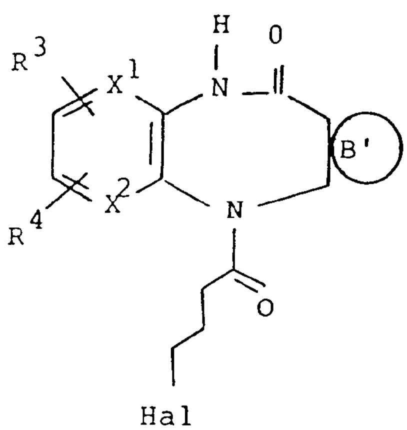

(II)

wherein

$R^3$, $R^4$, 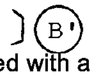 , $X^1$ and $X^2$ are defined as hereinbefore and Hal is a chlorine, bromine or iodine atom, is reacted with a secondary amine of general formula III

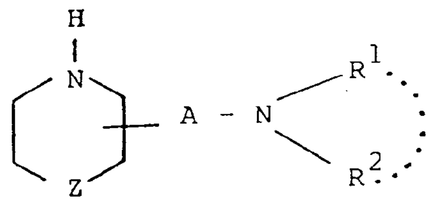

(III)

wherein
$R^1$, $R^2$, A and Z are defined as hereinbefore, in an inert solvent, preferably in the presence of an auxiliary base or an excess of the amine of general formula III, at temperatures of between -10°C and the boiling temperature of the reaction mixture or

b) in order to prepare base-substituted condensed diazepinones of general formula Ia in which the substituents are defined as hereinbefore, tricyclic compounds of general formula IV

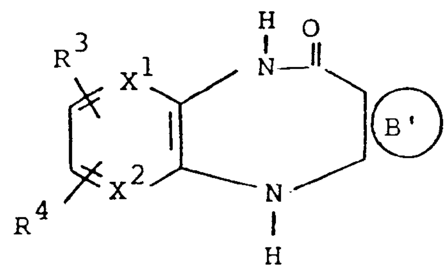

(IV)

wherein
the groups $R^3$, $R^4$, $X^1$, $X^2$ and 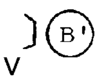 are defined as hereinbefore, are reacted with carboxylic acid derivatives of general formula V

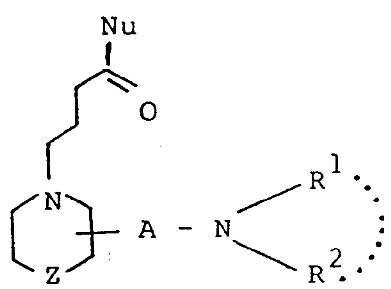

(V)

wherein
$R^1$, $R^2$, A and Z are defined as hereinbefore and Nu represents a nucleofugic group or leaving group, in an inert solvent at temperatures of between -25 and 130°C, optionally in the presence of a proton acceptor, or

c) in order to prepare a pyrrolo-condensed diazepinone of general formula Ib covered by general formula I,

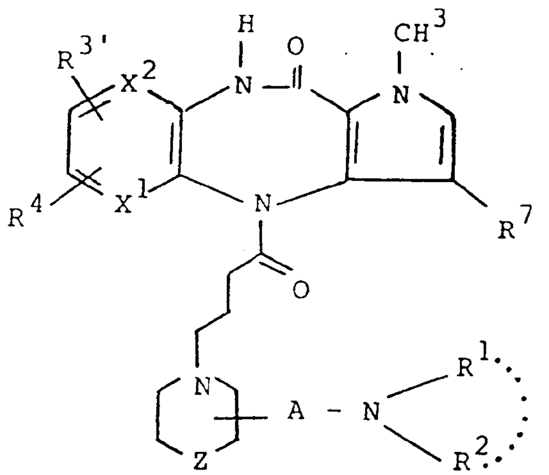

(Ib)

wherein
$R^1$, $R^2$, $R^3$, $X^1$, $X^2$ and A are defined as hereinbefore, Z has the meanings given hereinbefore with the exception of a sulphur atom, $R^3$, is an alkyl group having 1 to 4 carbon atoms or a hydrogen atom and $R^7$ represents a hydrogen atom,
a compound of general formula Ib wherein $R^7$ represents a chlorine atom is hydrogenolysed in a solvent with hydrogen, either in the presence of catalysts of metals of the VIIIth sub-group of the periodic Table of Elements under hydrogen pressures of from 1 to 300 bar and at temperatures of from 0°C to 130°C or with formic acid in the presence of palladium on charcoal as catalyst at temperatures of between 70 and 110°C or with triethylammonium formate in the presence of excess triethylamine and palladium on animal charcoal or palladium acetate and triarylphosphines, at temperatures of between 40 and 110°C
and if desired compounds of general formula I thus obtained are separated into their diastereoisomeric or enantiomeric forms and/or compounds of general formula I thus obtained are converted into their salts, preferably their physiologically acceptable salts with inorganic or organic acids.

2. Process as claimed in claim 1b, characterised in that acid halides, esters, anhydrides or mixed acid anhydrides are used as the carboxylic acid derivatives of general formula V.

## Revendications

### Revendications pour les Etats contractants suivants : AT, BE, CH, DE, FR, GB, IT, LU, NL, SE

1. Diazépinones condensées de formule générale I

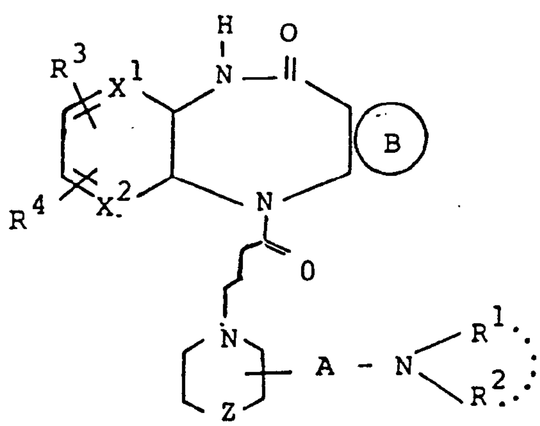

(I)

dans laquelle 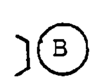 représente l'un des restes divalents

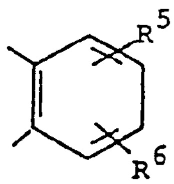 , 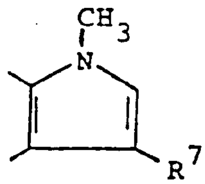 , 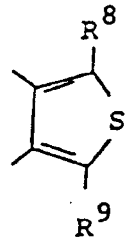 , 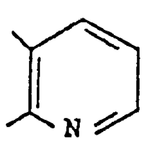 ou 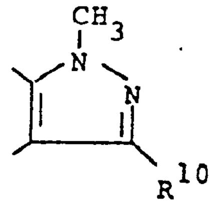

(S) (T) (U) (V) (W)

et

$X^1$i, $X^2$ A, $R^1$ à $R^{10}$ et Z ont les significations suivantes;

$X^1$ et $X^2$ représentent un groupe =CH ou, lorsque 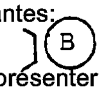 revêt les significations des restes divalents (S) , (U) ou (W) cités ci-dessus, peuvent aussi représenter tous les deux ou seulement $X^1$ ou seulement $X^2$ un atome d'azote;

A est un reste alkylène saturé linéaire ayant 1 à 6 atomes de carbone;

$R^1$ et $R^2$ sont des restes alkyle identiques ou différents ayant jusqu'à 6 atomes de carbone ou représentent avec l'atome d'azote situé entre eux un cycle hétéroaliphatique monocyclique, saturé, ayant 4 à 7 chaînons qui peut éventuellement être interrompu par un atome d'oxygène ou par le groupe N-$CH_3$;

$R^3$ est un groupe alkyle ayant 1 à 4 atomes de carbone, un atome de chlore ou un atome d'hydrogène;

$R^4$ est un atome d'hydrogène ou un groupe méthyle;

$R^5$ et $R^6$ représentent chacun un atome d'hydrogène, un atome de fluor, de chlore ou de brome ou un groupe alkyle ayant 1 à 4 atomes de carbone;

$R^7$ est un atome d'hydrogène ou de chlore ou un groupe méthyle;

$R^8$ est un atome d'hydrogène ou un reste alkyle ayant 1 à 4 atomes de carbone;

$R^9$ est un atome d'hydrogène ou un atome d'halogène ou un groupe alkyle ayant 1 à 4 atomes de carbone;

$R^{10}$ est un atome d'hydrogène ou un groupe méthyle et

Z est une liaison simple, un atome d'oxygène ou de soufre, le groupe méthylène ou 1,2-éthylène;

dans laquelle, lorsque 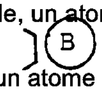 représente le reste divalent (T) et $R^7$ est un atomte d'hydrogène, $R^3$ ne peut pas représenter un atome de chlore et Z ne peut pas représenter un atome de soufre;

éventuellement leurs formes diastéréoisomères ou énantiomères et leurs sels, acceptables du point de vue physiologique, avec des acides inorganiques ou organiques.

2. Diazépinones condensées de formule générale I selon la revendication 1, dans laquelle

$X^1$ représente un groupe =CH, et

soit $X^2$ représente un atome d'azote et 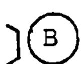 représente le reste divalent (S), à condition que $R^3$, $R^4$ et $R^5$ soient des atomes d'hydrogène et que $R^6$ soit un atome d'hydrogène, un atome de chlore ou un atome de brome, ou un groupe méthyle ou éthyle en huitième ou neuvième position de l'hétérocycle soit $X^2$ représente un groupe =CH lorsque 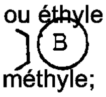 revêt la signification du reste divalent V ou U, $R^8$ étant un atome d'hydrogène et $R^9$ un groupe méthyle;

A représente une chaîne alkylène saturée non ramifiée ayant 1 à 5 atomes de carbone;

$R^1$ et $R^2$ représentent des restes alkyle identiques ou différents ayant 1 à 5 atomes de carbone ou représentent avec l'atome d'azote inclus un cycle hétéroaliphatique monocyclique, saturé, de 4 à 7 chaînons, qui peut éventuellement être interrompu par un atome d'oxygène ou par le groupe N-$CH_3$

et

Z représente une liaison simple, un atome d'oxygène, le groupe méthylène ou 1,2-éthylène et, éventuellement, leurs formes diastéréoisomères ou énantiomères, et leurs sels, acceptables du point de vue physiologique, avec des acides inorganiques ou organiques.

3. Diazépinones condensées de formule générale I selon les revendications 1 et 2, dans laquelle

$X^1$ représente un groupe =CH;

$X^2$ représente un atome d'azote et 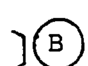 représente le reste divalent (S), à condition que $R^3$, $R^4$ et $R^5$ soient des atomes d'hydrogène et que $R^6$ soit un atome d'hydrogène, un atome de chlore ou un atome de brome, ou un groupe méthyle ou éthyle en huitième ou neuvième position de l'hétérocycle,

A représente une chaîne alkylène saturée non ramifiée ayant 1 à 5 atomes de carbone;

$R^1$ et $R^2$ représentent des restes alkyle identiques ou différents ayant 1 à 5 atomes de carbone ou représentent avec l'atome d'azote situé entre eux un cycle hétéroaliphatique monocyclique, saturé, de 5 à 7

chaînons, qui peut éventuellement être interrompu par un atome d'oxygène ou par le groupe N-$CH_3$ et
Z représente une liaison simple, un atome d'oxygène, le groupe méthylène ou 1,2-éthylène
et, éventuellement, leurs formes diastéréoisomères ou énantiomères, et leurs sels, acceptables du point de vue physiologique, avec des acides inorganiques ou organiques.

**4.** En tant que diazépinones condensées selon les revendications 1 à 3, les composés
11-[4-[3-[(diéthylamino)méthyl]-4-morpholinyl]-1-oxo-butyl]-5,11-dihydro-6H-pyrido[2,3-b][1,4]benzodiazépine-6-one;
11-[4-[2-[(diéthylamino)méthyl]-1-pipéridinyl]-1-oxo-butyl]-5,11-dihydro-9-méthyl-6H-pyrido[2,3-b][1,4]benzodiazépine-6-one;
5,11-dihydro-11-[4-[2-[(1-pirrolidinyl)méthyl]-1-pipéridinyl]-1-oxobutyl]-6H-pyrido[2,3-b][1,4]benzodiazépine-6-one
et leurs sels acceptables du point de vue physiologique avec des acides inorganiques ou organiques.

**5.** Médicament contenant un ou plusieurs composés selon les revendications 1 à 4 ainsi que des véhicules et/ou adjuvants habituels.

**6.** Utilisation des composés selon les revendications 1 à 4 pour la préparation de médicaments pour le traitement des bradycardies et des bradyarythmies et éventuellement des spasmes de la vessie et du côlon.

**7.** Procédé de préparation de diazépinones condensées de formule générale I selon la revendication 1, éventuellement de leurs formes diastéréoisomères ou énantiomères et de leurs sels avec des acides inorganiques ou organiques, caractérisé en ce que
a) pour la préparation de diazépinones condensées, substituées de manière basique, de formule générale Ia

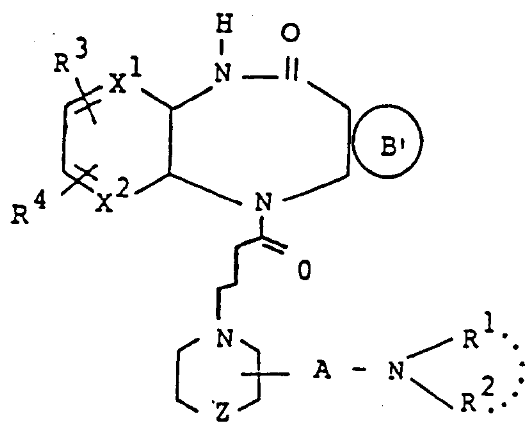

(Ia)

dans laquelle
$R^1$, $R^2$, $R^3$, $R^4$, $X^1$, $X^2$, A et Z ont les significations indiquées ci-dessus et 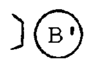 représente l'un des restes divalents (S), (U), (V), (W) ou (T')

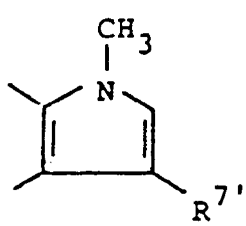

(T')

$R^{7'}$ étant un atome de chlore ou un groupe méthyle, un dérivé d'halogénoacyle de formule générale II

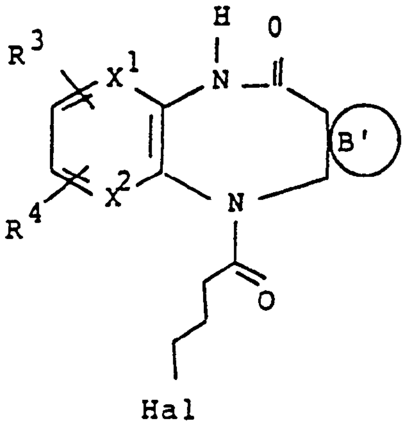

(II)

dans laquelle
$R^3$, $R^4$, 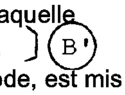 , $X^1$ et $X^2$ ont les significations indiquées et Hal représente un atome de chlore, de brome ou d'iode, est mis à réagir avec une amine secondaire de formule générale III

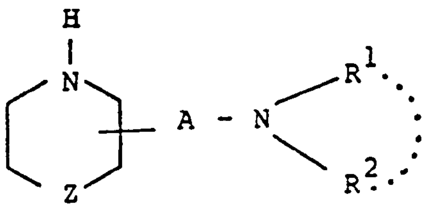

(III)

dans laquelle
$R^1$, $R^2$, A et Z sont définis comme ci-dessus, dans un solvant inerte, de préférence en présence d'une base auxiliaire ou d'un excès de l'amine de formule générale III, à des températures comprises entre -10°C et le point d'ébullition du mélange réactionnel, ou bien
b) pour la préparation de diazépinones condensées, substituées de manière basique, de formule générale Ia, avec les significations citées précédemment pour les substituants, des composés tricycliques de formule générale IV

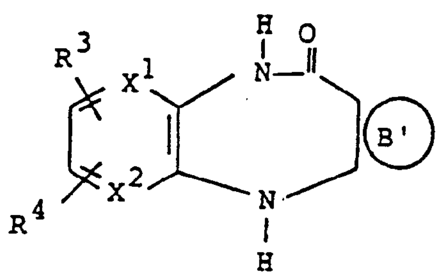

(IV)

dans laquelle
les restes $R^3$, $R^4$, $X^1$, $X^2$ et 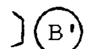 ont les significations indiquées ci-dessus, sont mis à réagir avec des dérivés d'acide carboxylique de formule générale V

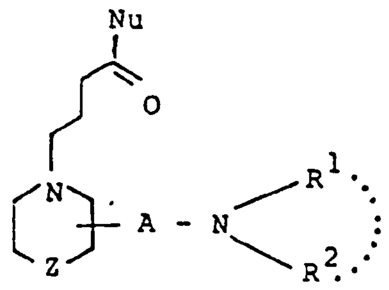

(V)

dans laquelle
$R^1$, $R^2$, A et Z sont définis comme ci-dessus, et Nu représente un groupe nucléofuge ou un groupe partant, dans un solvant inerte, à des températures comprises entre -25°C et 130°C, éventuellement en présence d'un accepteur de protons, ou bien
c) pour la préparation d'une diazépinone pyrrolo-condensée répondant à la formule générale I, de formule générale Ib

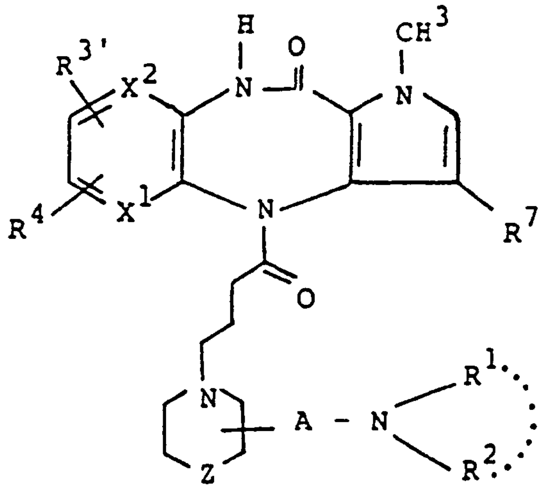

(Ib)

dans laquelle
$R^1$, $R^2$, $R^4$, $X^1$ , $X^2$ et A sont définis comme ci-dessus, Z a les significations indiquées ci-dessus à l'exception d'un atome de soufre, $R^{3'}$ représente un groupe alkyle ayant 1 à 4 atomes de carbone ou un atome d'hydrogène et $R^7$ représente un atome d'hydrogène, un tel composé de formule générale Ib dans laquelle $R^7$ représente un atome de chlore est hydrogénolysé dans un solvant soit en présence de catalyseurs de métaux du VIII$^{ème}$ sous-groupe du système périodique des éléments sous des pressions d'hydrogène de 1 à 300 bar et à des températures comprises entre 0 et 130°C soit avec l'acide formique en présence de palladium sur charbon comme catalyseur à des températures situées entre 70 et 110°C, soit avec le formiate de triéthylammonium en présence de triéthylamine en excès et de palladium sur noir animal ou d'acétate de palladium et de triarylphosphines, à des températures comprises entre 40 et 110°C
et, éventuellement, les composés de formule générale I ainsi obtenus sont résolus en leurs formes diastéréoisomères ou énantiomères et/ou les composés de formule générale I ainsi obtenus sont convertis en leurs sels, de préférence en leurs sels, acceptables du point de vue physiologique, avec des acides inorganiques ou organiques.

**8.** Procédé selon la revendication 7b, caractérisé en ce que l'on utilise comme dérivés d'acide carboxylique de formule générale V des halogénures, esters, anhydrides ou anhydrides d'acide mixtes.

**Revendications pour les Etats contractants suivants : ES, GR**

**1.** Procédé de préparation de diazépinones condensées de formule générale I

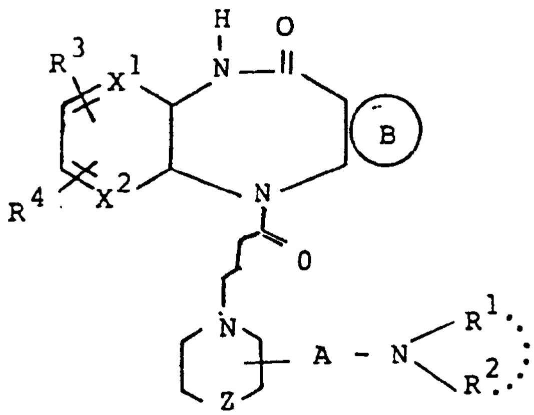

(I)

dans laquelle 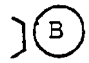 représente l'un des restes divalents

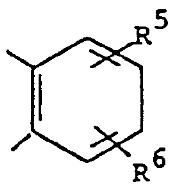 , 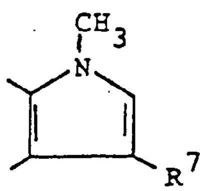 , 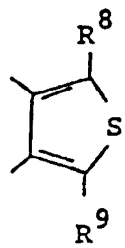 , 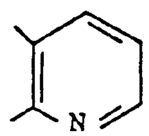 ou 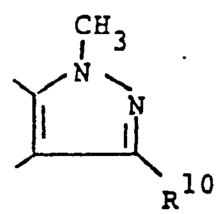

(S) (T) (U) (V) (W)

et

$X^1$, $X^2$, A, $R^1$ à $R^{10}$ et Z ont les significations suivantes:

$X^1$ et $X^2$ représentent un groupe =CH ou, lorsque 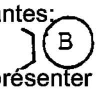 revêt les significations des restes divalents (S), (U) ou (W) cités ci-dessus, peuvent aussi représenter tous les deux ou seulement $X^1$ ou seulement $X^2$ un atome d'azote;

A est un reste alkylène saturé linéaire ayant 1 à 6 atomes de carbone;

$R^1$ et $R^2$ sont des restes alkyle identiques ou différents ayant jusqu'à 6 atomes de carbone ou représentent avec l'atome d'azote situé entre eux un cycle hétéroaliphatique monocyclique, saturé, ayant 4 à 7 chaînons qui peut éventuellement être interrompu par un atome d'oxygène ou par le groupe $N-CH_3$;

$R^3$ est un groupe alkyle ayant 1 à 4 atomes de carbone, un atome de chlore ou un atome d'hydrogène;

$R^4$ est un atome d'hydrogène ou un groupe méthyle;

$R^5$ et $R^6$ représentent chacun un atome d'hydrogène, un atome de fluor, de chlore ou de brome ou un groupe alkyle ayant 1 à 4 atomes de carbone;

$R^7$ est un atome d'hydrogène ou de chlore ou un groupe méthyle;

$R^8$ est un atome d'hydrogène ou un reste alkyle ayant 1 à 4 atomes de carbone;

$R^9$ est un atome d'hydrogène ou un atome d'halogène ou un groupe alkyle ayant 1 à 4 atomes de carbone;

$R^{10}$ est un atome d'hydrogène ou un groupe méthyle et

Z est une liaison simple, un atome d'oxygène ou de soufre, le groupe méthylène ou 1,2-éthylène;

dans laquelle, lorsque 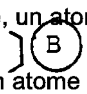 représente le reste divalent (T) et $R^7$ est un atome d'hydrogène, $R^3$ ne peut pas représenter un atome de chlore et Z ne peut pas représenter un atome de soufre;

éventuellement de leurs formes diastéréoisomères ou énantiomères et de leurs sels avec des acides inorganiques ou organiques, caractérisé en ce que

a) pour la préparation de diazépinones condensées, substituées de manière basique, de formule générale Ia

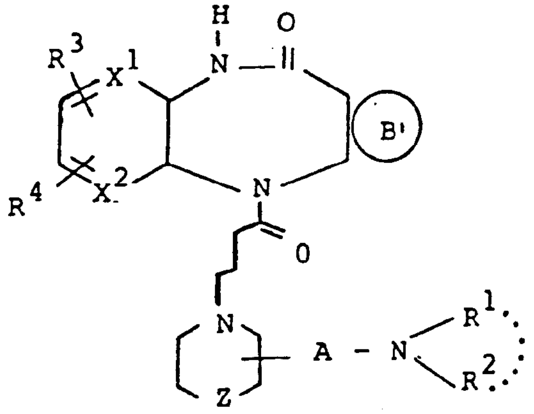

(Ia)

dans laquelle
$R^1$, $R^2$, $R^3$ $R^4$, $X^1$, $X^2$, A et Z ont les significations indiquées ci-dessus et 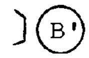 représente l'un des restes divalents (S), (U), (V), (W) ou (T')

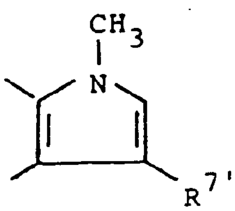

(T')

$R^{7'}$ étant un atome de chlore ou un groupe méthyle, un dérivé d'halogénoacyle de formule générale II

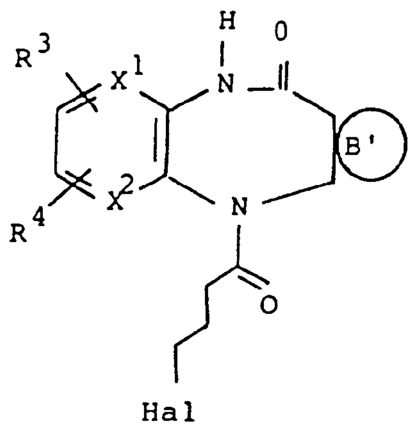

(II)

dans laquelle
$R^3$, $R^4$, 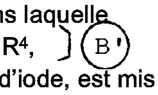 , $X^1$ et $X^2$ ont les significations indiquées et Hal représente un atome de chlore, de brome ou d'iode, est mis à réagir avec une amine secondaire de formule générale III

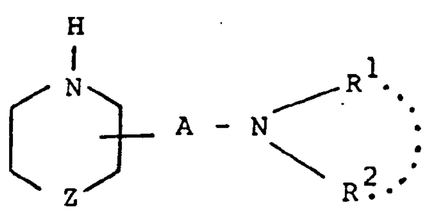

(III)

dans laquelle
$R^1$, $R^2$, A et Z sont définis comme ci-dessus, dans un solvant inerte, de préférence en présence d'une

base auxiliaire ou d'un excès de l'amine de formule générale III, à des températures comprises entre -10°C et le point d'ébullition du mélange réactionnel, ou bien

b) pour la préparation de diazépinones condensées, substituées de manière basique, de formule générale Ia, avec les significations citées précédemment pour les substituants, des composés tricycliques de formule générale IV

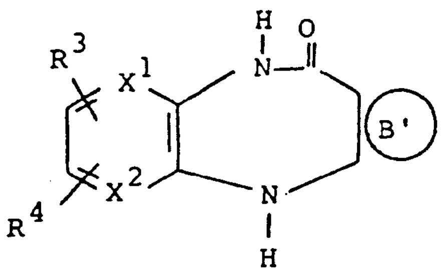

(IV)

dans laquelle

les restes $R^3$, $R^4$, $X^1$, $X^2$ et 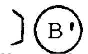 ont les significations indiquées ci-dessus, sont mis à réagir avec des dérivés d'acide carboxylique de formule générale V

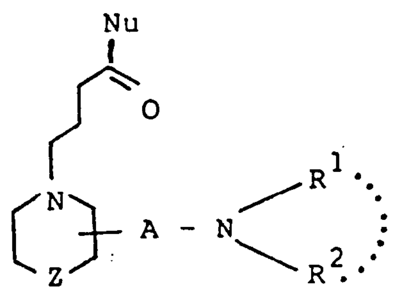

(V)

dans laquelle

$R^1$, $R^2$, A et Z, sont définis comme ci-dessus, et Nu représente un groupe nucléofuge ou un groupe partant, dans un solvant inerte, à des températures comprises entre -25°C et 130°C, éventuellement en présence d'un accepteur de protons, ou bien

c) pour la préparation d'une diazépinone pyrrolo-condensée répondant à la formule générale I, de formule générale Ib

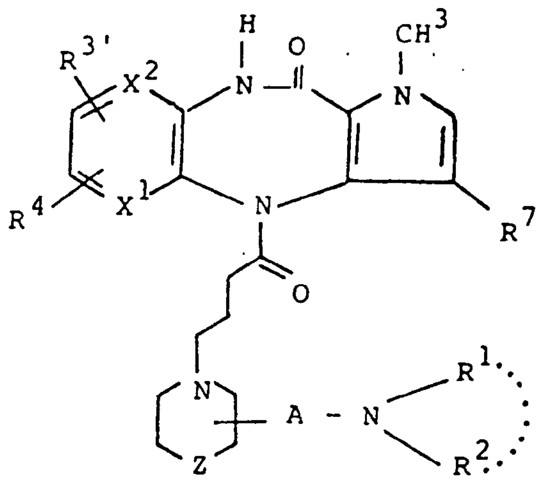

(Ib)

dans laquelle

$R^1$, $R^2$, $R^4$, $X^1$, $X^2$ et A sont définis comme ci-dessus, Z a les significations indiquées ci-dessus à l'exception d'un atome de soufre, $R^{3'}$ représente un groupe alkyle ayant 1 à 4 atomes de carbone ou un atome d'hy-

drogène et $R^7$ représente un atome d'hydrogène, un tel composé de formule générale Ib dans laquelle $R^7$ représente un atome de chlore est hydrogénolysé dans un solvant soit en présence de catalyseurs de métaux du VIII$^{ème}$ sous-groupe du système périodique des éléments sous des pressions d'hydrogène de 1 à 300 bar et à des températures comprises entre 0 et 130°C soit avec l'acide formique en présence de palladium sur charbon comme catalyseur à des températures situées entre 70 et 110°C, soit avec le formiate de triéthylammonium en présence de triéthylamine en excès et de palladium sur noir animal ou d'acétate de palladium et de triarylphosphines, à des températures comprises entre 40 et 110°C
et, éventuellement, les composés de formule générale I ainsi obtenus sont résolus en leurs formes diastéréoisomères ou enantiomères et/ou les composés de formule générale I ainsi obtenus sont convertis en leurs sels, de préférence en leurs sels, acceptables du point de vue physiologique, avec des acides inorganiques ou organiques.

**2.** Procédé selon la revendication 1b, caractérisé en ce que l'on utilise comme dérivés d'acide carboxylique de formule générale V des halogénures, esters, anhydrides ou anhydrides d'acide mixtes.